# EUROPEAN PATENT SPECIFICATION

(11) **EP 1 381 272 B1**
(45) Date of publication and mention of the grant of the patent: **29.04.2009**
(21) Application number: 02719374.7
(22) Date of filing: 26.03.2002
(51) Int. Cl.: C12Q 1/68

(54) **EFFICIENT METHODS FOR ISOLATING FUNCTIONAL G-PROTEIN COUPLED RECEPTORS AND IDENTIFYING ACTIVE EFFECTORS AND EFFICIENT METHODS TO ISOLATE PROTEINS INVOLVED IN OLFACTION AND EFFICIENT METHODS TO ISOLATE AND IDENTIFYING ACTIVE EFFECTORS**
EFFIZIENTE VERFAHREN ZUR ISOLIERUNG FUNKTIONELLER G-PROTEIN-GEKOPPELTER REZEPTOREN UND IDENTIFIZIERUNG AKTIVER EFFEKTOREN UND EFFIZIENTE VERFAHREN ZUR ISOLIERUNG AN DER GERUCHSWAHRNEHMUNG BETEILIGTER PROTEINE SOWIE EFFIZIENTE VERFAHREN ZUR ISOLIERUNG UND IDENTIFIZIERUNG AKTIVER EFFEKTOREN
PROCEDES EFFICACES DESTINES A ISOLER DES RECEPTEURS FONCTIONNELS COUPLES A LA PROTEINE G ET A IDENTIFIER DES EFFECTEURS ACTIFS, DES PROCEDES EFFICACES DESTINES A ISOLER DES PROTEINES IMPLIQUEES DANS L'ODORAT ET DES PROCEDES EFFICACES DESTINES A ISOLER ET A IDENTIFIER DES EFFECTEURS ACTIFS

(30) Priority: 27.03.2001 US 279168 P; 31.01.2002 US 353392 P
(43) Date of publication of application: 21.01.2004
(73) Proprietor: Inscent, Inc., Irvine, CA 92614 (US)
(72) Inventor: WOODS, Daniel, Inscent, Inc., Irvine, CA 92614 (US); DIMITRATOS, Spiros, Inscent, Inc., Irvine, CA 92614 (US)
(74) Representative: Breese, Pierre
(86) International application number: PCT/US2002/009559
(87) International publication number: WO 2002/077200

(56) References cited:
- WO-A-01/14554
- WO-A-01/68805
- US-A- 2 026 393
- US-A- 3 828 104
- KRAUTWURST D ET AL: "Identification of ligands for olfactory receptors by functional expression of a receptor library" CELL, CELL PRESS, CAMBRIDGE, NA, US, vol. 95, 25 June 1998 (1998-06-25), pages 917-926, XP002153217 ISSN: 0092-8674
- KIM MIN-SU ET AL: "LUSH odorant-binding protein mediates chemosensory responses to alcohols in Drosophila melanogaster" GENETICS, vol. 150, no. 2, October 1998 (1998-10), pages 711-721, XP002342911 ISSN: 0016-6731
- DULAC CATHERINE: "Sensory coding of pheromone signals in mammals" CURRENT OPINION IN NEUROBIOLOGY, vol. 10, no. 4, August 2000 (2000-08), pages 511-518, XP002342912 ISSN: 0959-4388
- AFSHAR M. ET AL.: 'Towards structural models of molecular recognition in olfactory receptors' BIOCHIMIE vol. 80, 1998, pages 129 - 135, XP002955109
- AMREIN H. ET AL.: 'Genes expressed in neurons of adult male drosophila' CELL vol. 88, 21 February 1997, pages 459 - 469, XP002955110
- OHBAYASHI F. ET AL.: 'A homologue of the drosophila doublesex gene is transcribed into sec-specific mRNA isoforms in the silkworm, bombyx mori' COMP. BIOCHEM. PHYS. PART B vol. 128, 2001, pages 145 - 158, XP002955111

### SEQUENCE LISTING

<110> Inscent, Inc.
   Woods, Daniel
   Dimitratos, Spiros
<120> EFFICIENT METHODS FOR ISOLATING FUNCTIONAL G-PROTEIN COUPLED RECEPTORS AND IDENTIFYING ACTIVE EFFECTORS AND EFFICEINT METHODS TO ISOLATE PROTEINS INVOVLVED IN OLFACTION AND IDENTIFYING ACTIVE EFFECTORS
<130> INS-00101.P.1.1
<150> 60/279,168
   <151> 2001-03-27
<150> 60/353,392
   <151> 2002-01-31
<160> 6
<170> PatentIn version 3.1
<210> 1
   <211> 15
   <212> DNA
   <213> Artificial Sequence
<220>
   <223> Synthetic Construct
<400> 1
   tttttttttt ttttt 15
<210> 2
   <211> 22
   <212> DNA
   <213> Artificial Sequence
<220>
   <223> Synthetic Construct
<400> 2
   aaaaaaaaaa aaaactgtac cg 22
<210> 3
   <211> 22
   <212> DNA
   <213> Artificial Sequence
<220>
   <223> Synthetic Construct
<400> 3
   tttttttttt tttttgacat gg 22
<210> 4
   <211> 14
   <212> DNA
   <213> Artificial Sequence
<220>
   <223> Synthetic Constrcut
<400> 4
   aaaaaaaaaa aaaa 14
<210> 5
   <211> 15
   <212> DNA
   <213> Artificial Sequence
<220>
   <223> Synthetic Construct
<400> 5
   tttttttttt ttttt 15
<210> 6
   <211> 23
   <212> DNA
   <213> Artificial Sequence
<220>
   <223> Synthetic Construct
<220>
   <221> misc_feature
   <222> (16)..(23)
   <223> "n" can be any nucleotide
<400> 6
   tttttttttt tttttnnnnn nnn 23

## Description

### Technical Field

The present disclosure generally relates to methods and compositions for identifying and isolating G-protein coupled receptors (GPCRs), odorant binding proteins (OBPs), and other olfactory or neuronal proteins from any species where these proteins are expressed. The present disclosure also relates to methods for isolating either natural, synthesized or partially synthesized proteins or chemicals that interact with GPCRs, OBPs, or other olfactory proteins when evaluated in either a cell-based or non-cell based assay. Methods facilitating the *in vivo* evaluation of synthesized proteins or chemicals for interaction with olfactory proteins are also provided. Furthermore, the present disclosure provides the methods and means desirable to rapidly and efficiently array transcripts from cDNA libraries, including but not limited to a tissue-specific cDNA library, and to normalize the clones with respect to frequency of representation. The technologies presented herein are useful in a broad range of applications beyond isolating olfactory molecules, and are particularly useful when screening complex cDNA libraries for specific transcripts that are novel or unattainable using conventional methods based on homology to known forms or variants.

### Background

The identification and isolation of novel members of large protein families has traditionally been based on homology in primary and secondary structure between members of the family. That is, new members of a protein family have usually been identified based on the homology of their DNA sequence and amino acid sequence to known family members. This approach can be problematic when attempting to identify novel members of protein families when the existing members do not exhibit DNA or amino acid sequence conservation. For example, until recently efforts to identify insect odorant receptors, members of the G-Protein Coupled Receptor family, were fruitless because the genes encoding these receptors have generally not maintained sequence homology throughout evolution. Efforts have been made to identify related proteins by using structural inferences, which are typically more global properties. However, these efforts have also had limited success, perhaps because of the complex mathematical algorithms involved. Here, we describe a new method for identifying novel G-Protein Coupled Receptors specifically, and novel members of protein families with little known homology to existing members in general, based on differential tissue expression or interaction with specific protein or chemical constructs.

G-Protein Coupled Receptors (GPCRs) are a sequence-diverse group of integral membrane proteins with seven hydrophobic domains; they are therefore called Seven-Transmembrane or Serpentine Proteins. Their main role is the transmission of signals from the outside of the cell (typically a nerve cell or neuron) to the inside, via interaction with an external agonist or antagonist and an internal protein/ion/effector pathway involving trimeric G proteins such as Gα. GPCRs provide a means to achieve a cellular response to stimuli including taste, light, and odor, and are thus important for an organism's sensory perception of the environment. Since GPCRs are a diverse group of proteins, few of the results obtained by studying the structural properties of known members can be reliably extrapolated to novel members. Previous attempts to identify novel GPCRs have focused on the general property of seven hydrophobic transmembrane domains, but the current understanding of protein folding mechanics only allows inferences to be made. As a result, it has been difficult to identify novel GPCRs up until now.

There is therefore a need to develop effective methodologies and compositions for identifying novel G-Protein Coupled Receptors such as those described herein. These new approaches overcome the limitations of traditional approaches by not relying on sequence homology but rather on function and on protein-protein interactions. Consequently, the methods and compositions described here can be applied to the rapid identification and isolation of novel members of any protein family. They also permit the identification and isolation of proteins or chemicals that interact with the newly discovered proteins.

The article of Min-SU Kim et al. (Genetics, 1998, vol. 150, pages 711-721) discloses a Drosophila mutant, *lush,* with odorant-specific defects in olfactory behavior.

The US patent US 3,828,104 describes compositions useful in connection with the dissipation of malodorous materials present in the air of enclosed spaces and an aerosol spray useful as a space deodorant.

### Brief Description of the Figures

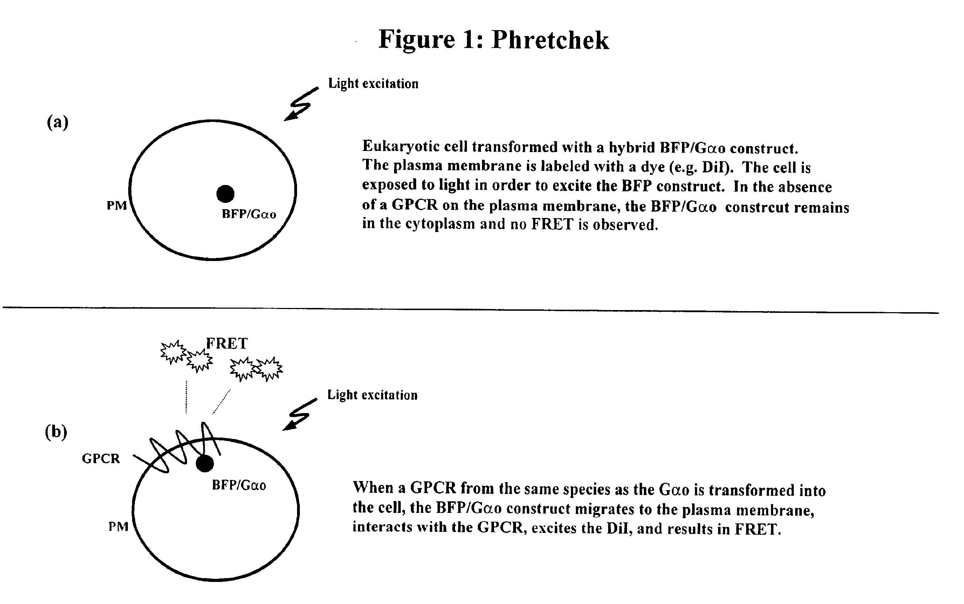

**FIG. 1****.** Diagram of the Phretcheck technique, which uses fluorescence to identify GPCRs. (a) Hybrid constructs containing DNA sequences encoding the C-terminus of the Gαo protein from the species studied fused to an indicator such as Blue Fluorescent Protein or BFP is transformed into cells (for example, yeast or *Drosophila*). When expressed, this construct can yield a hybrid BFP/Gαo protein localized in the cytoplasm. The cell's plasma membranes are labeled with DiI or another indicator capable of FRET when in contact with the BFP/Gαo fusion protein, and the cells are exposed to light of a wavelength that would result in FRET if the DiI and BFP are in contact. However, in the absence of a GPCR, the BFP/Gαo fusion protein remains in the cytoplasm, where it cannot interact with the DiI in the plasma membrane. Thus, no FRET is observed in the absence of a GPCR. (b) Cells are transformed with a library of GPCR candidate molecules. Cells expressing a GPCR in their plasma membranes can be identified by the FRET resulting from the interaction of the GPCR with the BFP/Gαo fusion protein. PM = Plasma membrane; BFP/Gαo = Blue Fluorescent Protein - Gαo fusion construct; GPCR = G-protein coupled receptor; FRET = Fluorescent Resonance Energy Transfer.

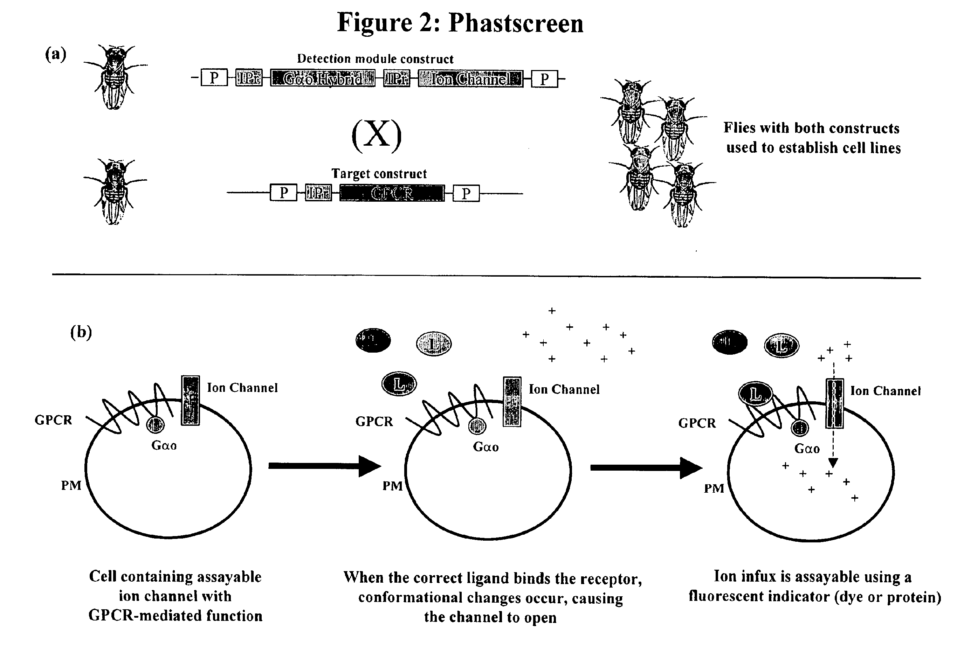

**FIG. 2****.** Diagram of Phastscreen, a cell-based assay for determining if a ligand is capable of binding a GPCR The Phastscreen technique is capable of identifying lead compounds that can be developed into Arometics. (a) Two different strains of *Drosophila,* one transformed with the detection module and the other transformed with the target construct, are crossed to create a strain carrying both constructs. The general DNA sequence of the constructs is depicted. Tissue is isolated from these animals and used to establish cell lines. (b) A cell expressing both the detection module and target construct is exposed to potential ligands. Upon binding, the ligand activates the GPCR, which causes an influx of ions. The ion influx produces an emission from the fluorescent indicator. P = P-element repeat, a sequence characteristic of *Drosophila* transposable P-elements; IPr = Inducible promoter sequence; Gαo = subunit of trimeric G-protein complex that interacts with G-protein coupled receptors in the presence of a ligand; GPCR = G-protein coupled receptor, a specialized seven-transmembrane or serpentine receptor active in the odor reception pathway; Ion channel = a protein or complex embedded in the plasma membrane and whose activity is regulated by the trimeric G-protein. An ion channel selectively allows ions to cross the membrane; L = ligand, with the different colors signifying different tested compounds, only one of which is able to bind the GPCR and activate the signaling cascade; PM = plasma membrane; + = ion with positive charge.

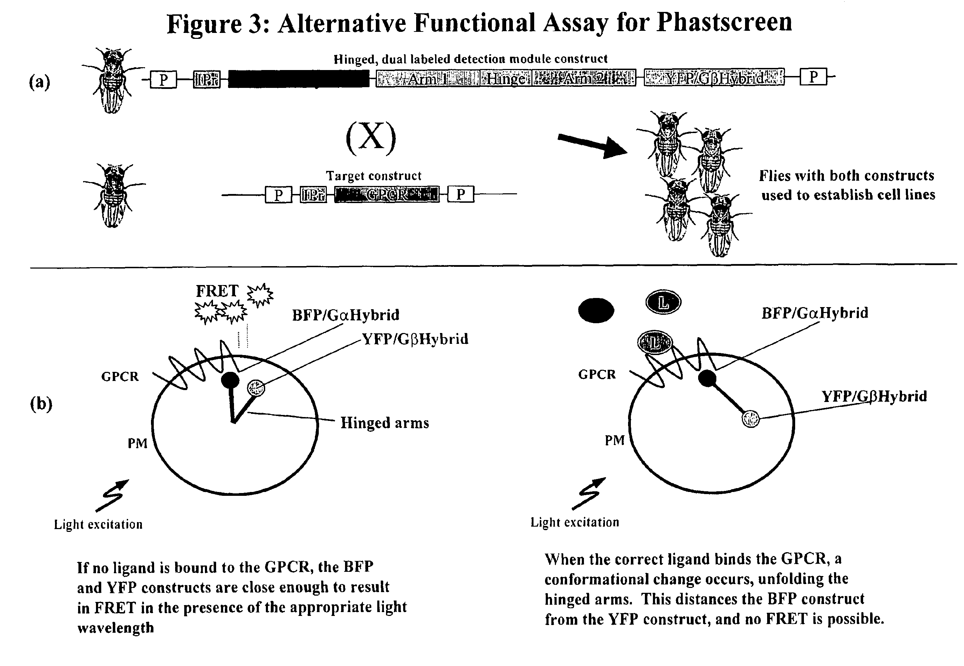

**FIG. 3****.** An alternative *Phastscreen* assay. This methodology takes advantage of the dissociation between Gαo and Gβ that occurs only when the G-protein complex interacts with an activated GPCR, that is, a GPCR with a ligand bound to it. (a) A DNA construct encoding a BFP/Gαo hybrid and an YFP/Gβ hybrid connected by flexible polypeptide motifs (the "arms" and "hinge" sections depicted) is transformed into a *Drosophila* strain using P-element mediated transformation. These flies are crossed with another strain that has been transformed with a target construct encoding a GPCR. The progeny with both constructs are isolated and their tissues used to establish cell lines. (b) In the cell, the BFP/Gαo and YFP/Gβ hybrids are in proximity, resulting in FRET when the cell is exposed to light of the appropriate wavelength. However, if the GPCR is bound by a ligand and activated, the YFP/Gβ hybrid dissociates from the BFP/Gαo hybrid, and no FRET is observed. Thus, ligands can be assayed for their ability to bind the GPCR, and this Phretcheck method can be used to identify potential Arometics. P = P-element repeat; IPr = Inducible promoter sequence; PM = Plasma membrane; BFP/ Gαo = Blue Fluorescent Protein - Gαo fusion construct; YFP/ Gβ = Blue Fluorescent Protein - Gβ fusion construct; GPCR = G-protein coupled receptor; FRET = Fluorescent Resonance Energy Transfer; L = ligand, with the different colors signifying different tested compounds, only one of which is able to bind the GPCR and activate the signaling cascade; PM = plasma membrane.

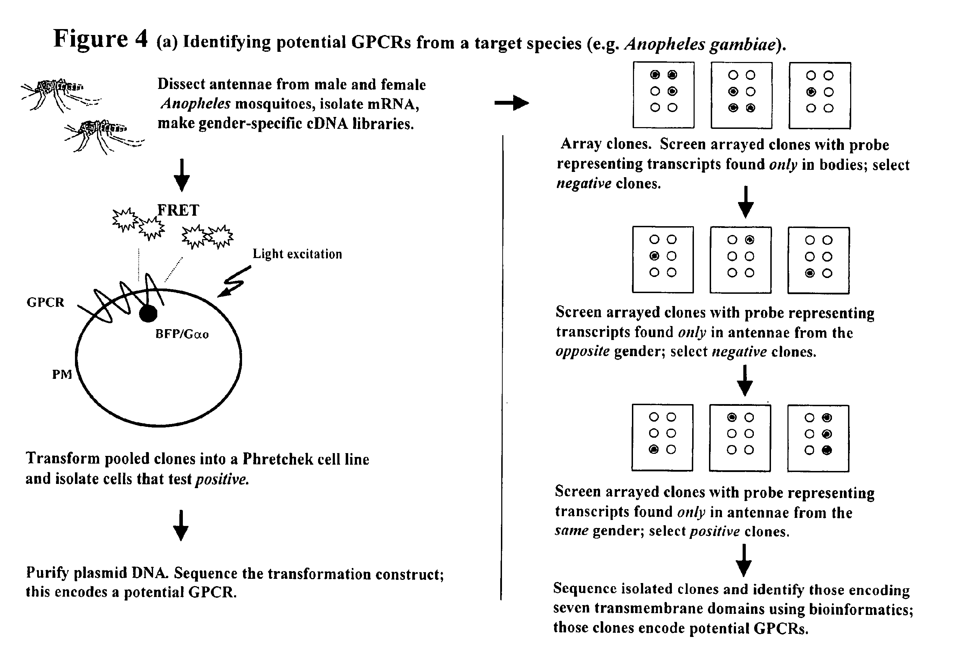

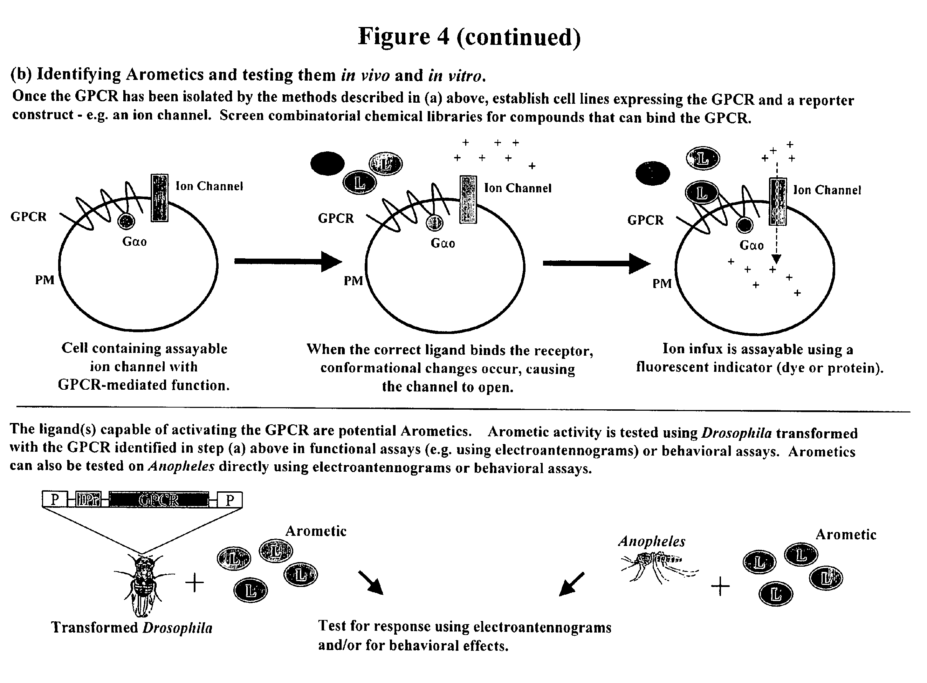

**FIG. 4****.** Outline of a procedure to isolate a pheromone odorant receptor using the methods provided by the present disclosure. *Anopheles gambiae* is used as an example but the same general approach can be applied to other species. (a) First, potential GPCRs from the target species must be identified. Antennae from male and female mosquitoes are dissected and used to generate gender-specific cDNA libraries, that is, libraries representing transcripts expressed specifically in the antennae of one gender or the other. One method to isolate GPCRs is to use Phretchek cells transformed with clones from the libraries (left). Cells that test positive under Phretchek have been transformed with constructs encoding potential GPCRs. Plasmid DNA is purified from these cells and the construct(s) sequenced. Another method to isolate GPCRs involves screening clones and selecting only those clones expressed in the antennae of male mosquitoes (right), since the males are attracted to pheromone emitted by females. The libraries are arrayed and subjected to a series of screens designed to initially identify those clones that are expressed specifically in the antennae, then to isolate those clones that are expressed only in the antennae of one gender and not the other. Once these clones have been isolated, they are sequenced. Bioinformatic analysis of the DNA sequences can identify those clones encoding seven transmembrane domains; these encode potential GPCRs. (b) Arometics can be developed from compounds able to activate the GPCR. Cell lines expressing the GPCR and a reporter cascade can be screened using the *Phastscreen* method provided by the present disclosure. In this illustration, an ion channel reporter construct is used (top). Cells are exposed to a large variety of potential ligands by screening combinatorial chemical libraries. Ligands capable of activating the GPCR will result in an assayable ion flux across the plasma membrane, are potential Arometics, and in this example act as agonists. *Arometic* activity is tested *in vivo* and *in vitro* (bottom). *Drosophila* transformed with the *Anopheles* GPCR are tested for behavioral response to the *Arometic in vivo.* Antennae from *Drosophila* transformed with the *Anopheles* GPCR are dissected and used in electroantennograms to determine whether the *Arometic* is capable of activating the GPCR. The *Arometic* is also tested using *Anopheles* in behavioral assays, and using electroantennograms with dissected *Anopheles* antennae. P = P-element repeat; IPr = Inducible promoter sequence; BFP/ Gαo = Blue Fluorescent Protein - Gαo fusion construct; Gαo = subunit of trimeric G-protein complex that interacts with G-protein coupled receptors; GPCR = G-protein coupled receptor; FRET = Fluorescent Resonance Energy Transfer; L = ligand, with the different colors signifying different tested compounds, only one of which is able to bind the GPCR and activate the signaling cascade; PM = plasma membrane; + = ion with positive charge.

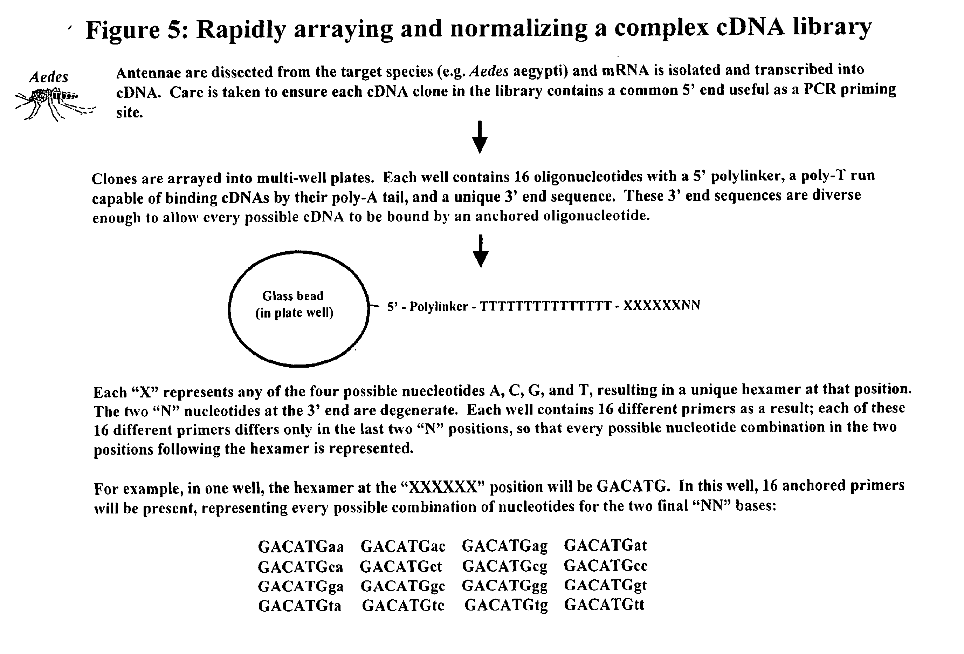

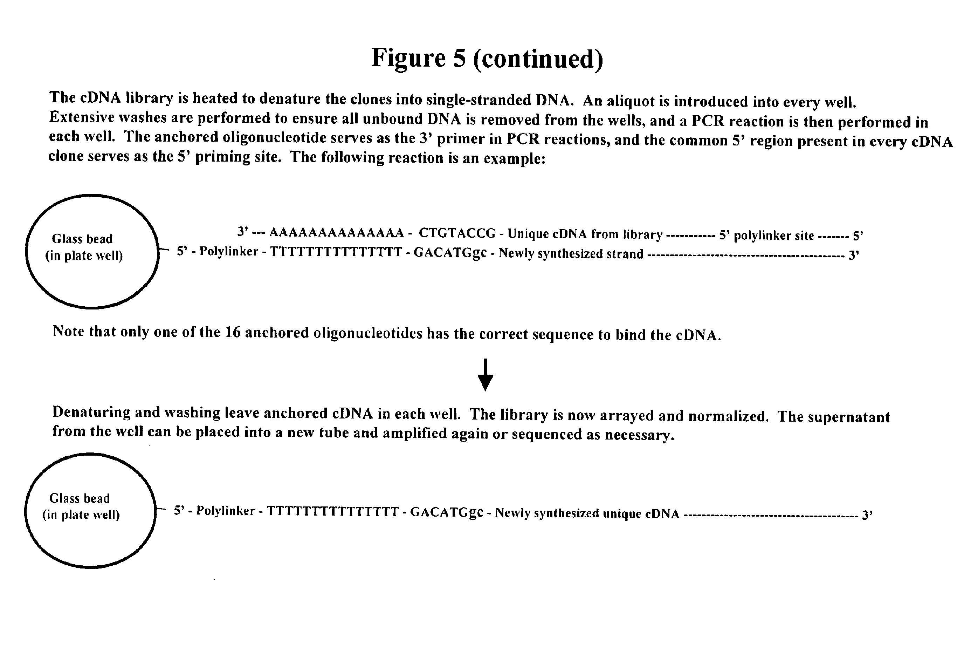

**FIG. 5****.** A method for rapidly arraying and normalizing a complex cDNA library. Antennae are dissected from the target species and mRNA is purified and transcribed into cDNA. Each clone in the library is designed to contain a common 5' end that will be useful as a PCR priming site subsequently. In this method, multi-well plates with each well containing 16 oligonucleotides with a 5'polylinker, a poly-T run, and a unique 3' end sequence are used. The cDNA clones are arrayed into these multi-well plates. The 3' end sequences found in the wells are diverse enough to allow every possible cDNA to be bound by an anchored oligonucleotide. The library is heated to denature the clones, and PCR reactions are performed in each well. Denaturation and wash steps leave anchored cDNA in each well, and the library is now arrayed and normalized.

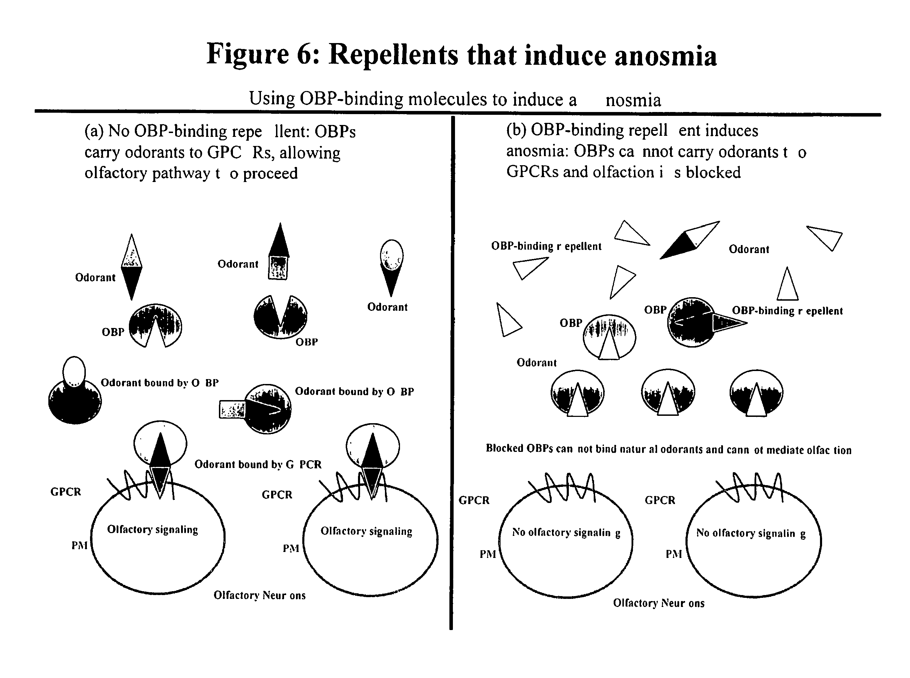

**FIG. 6****.** A model of novel repellent function based on inducing anosmia. Our novel repellents are based on molecules identified from screening combinatorial chemical libraries. These molecules are selected for their ability to bind OBPs and render them unable to bind odorants. (a) Hydrophobic odorants enter the hemolymph of mosquito antennal tissue and are bound by OBPs that transport them through the hydrophilic medium to the surface of olfactory neurons, where the OBPs are bound by GPCRs. This initiates the olfactory signaling cascade and results in a behavioral response from the mosquito. (b) In the presence of OBP-binding repellent molecules, OBPs cannot bind natural odorants and the olfactory signaling cascade is blocked. Thus, repellents based on OBP-binding molecules induce anosmia. Alternatively, the OBP-binding molecules can be used to generate attractants, providing the OBP these molecules interact with is involved in an olfactory pathway that results in the target species being attracted to a scent.

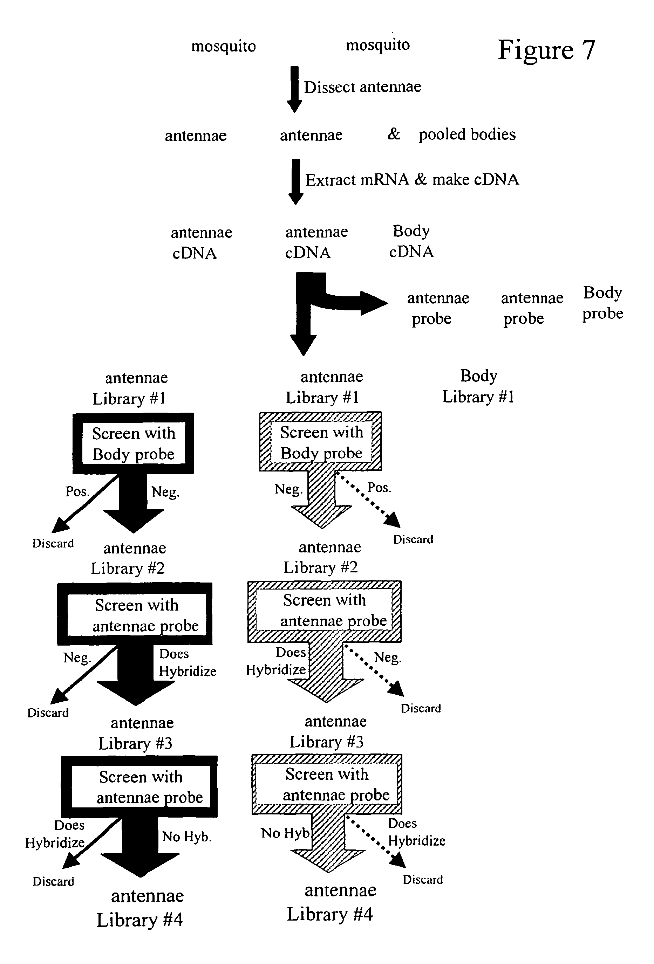

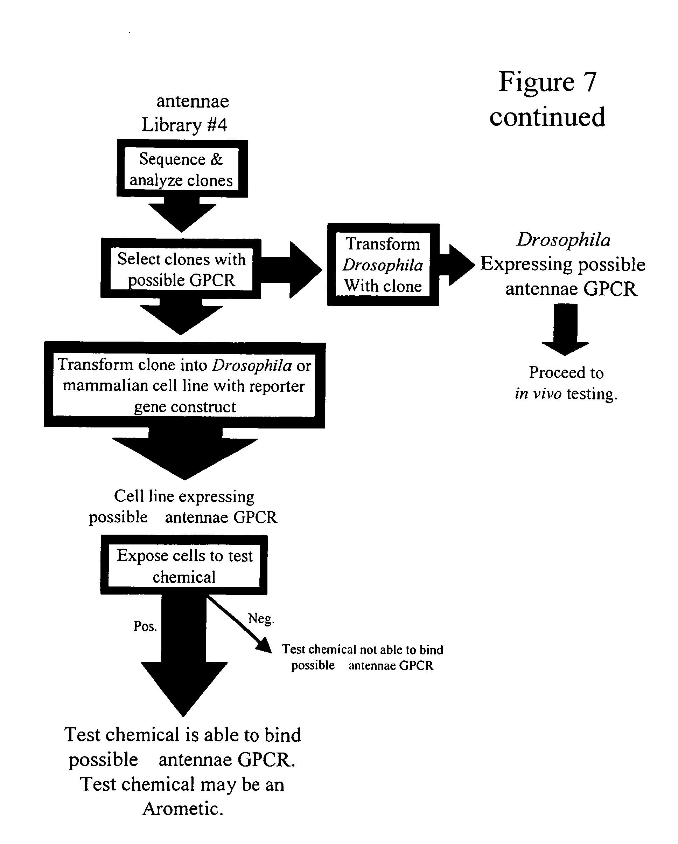

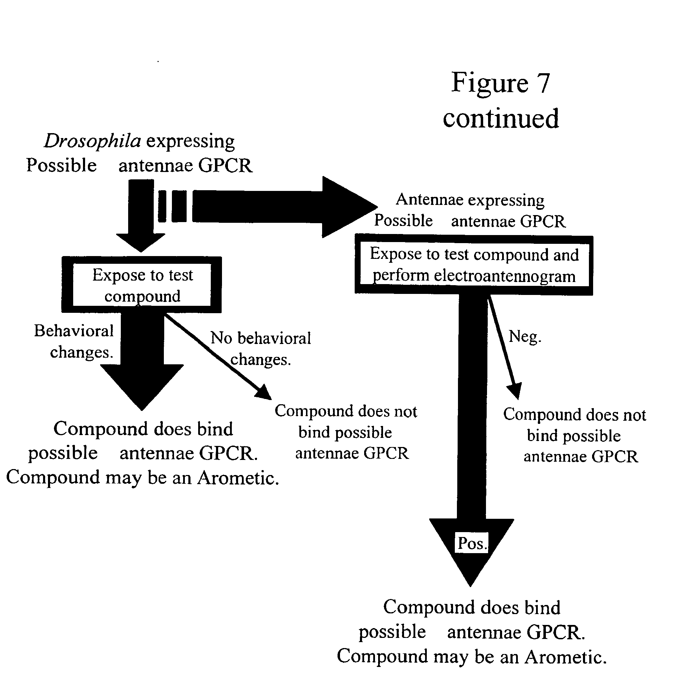

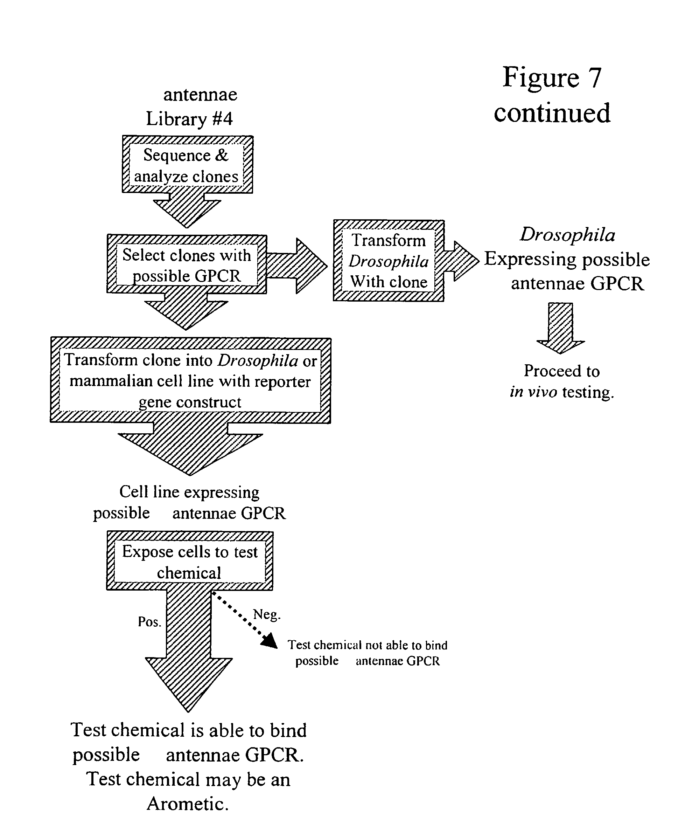

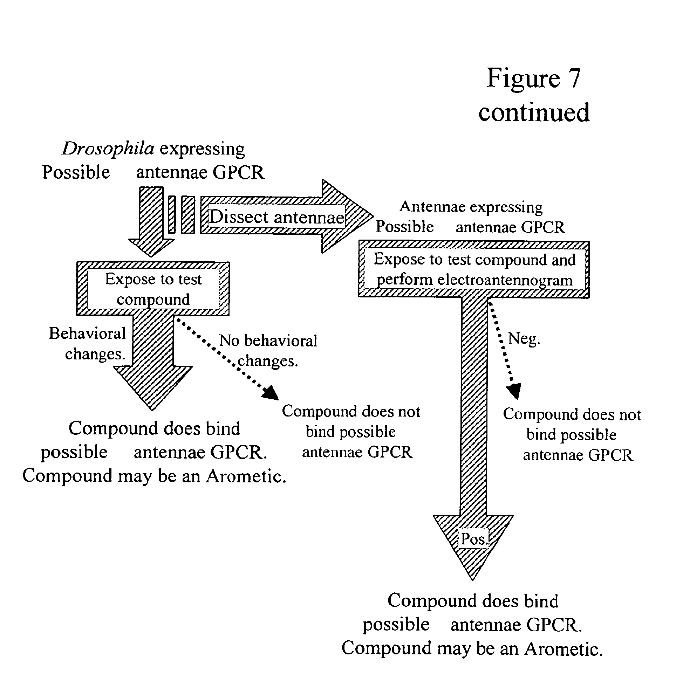

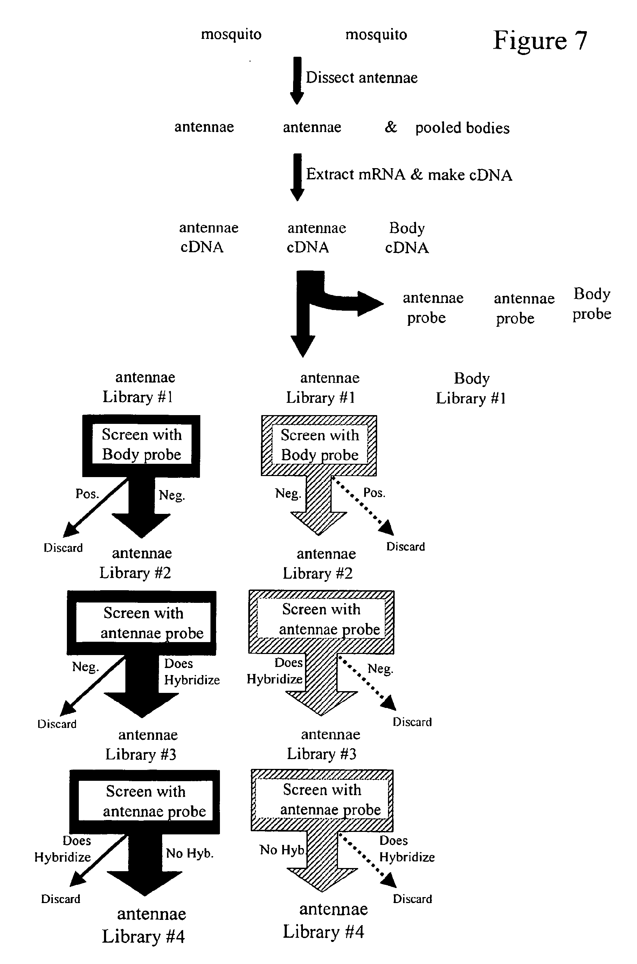

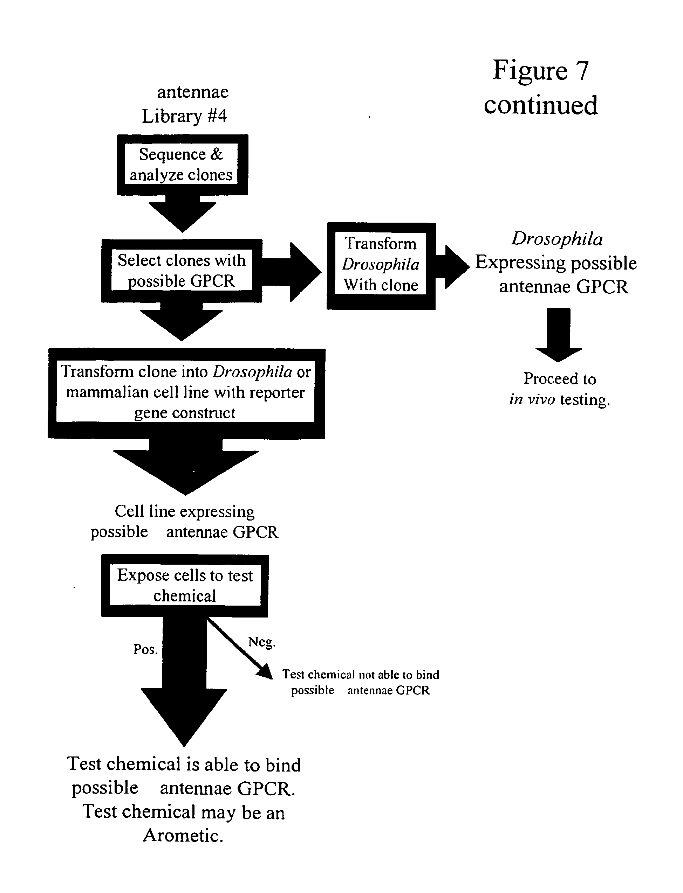

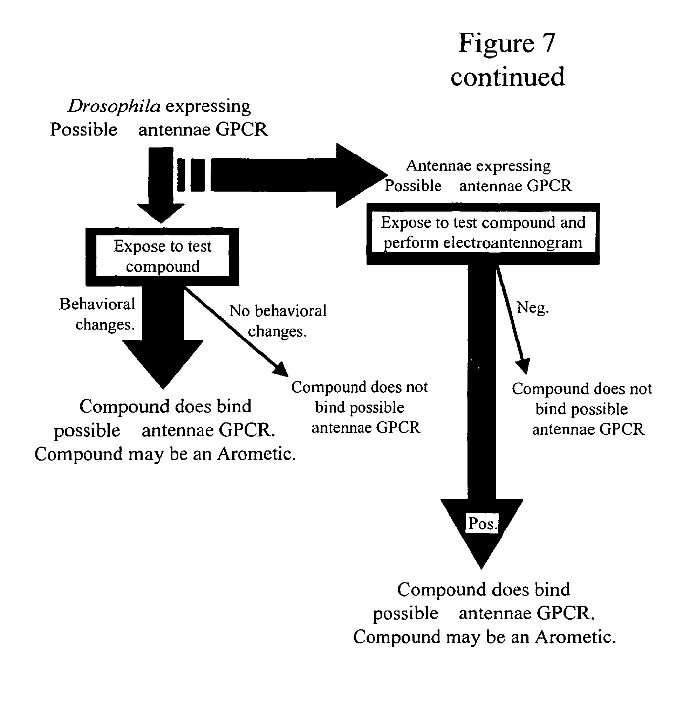

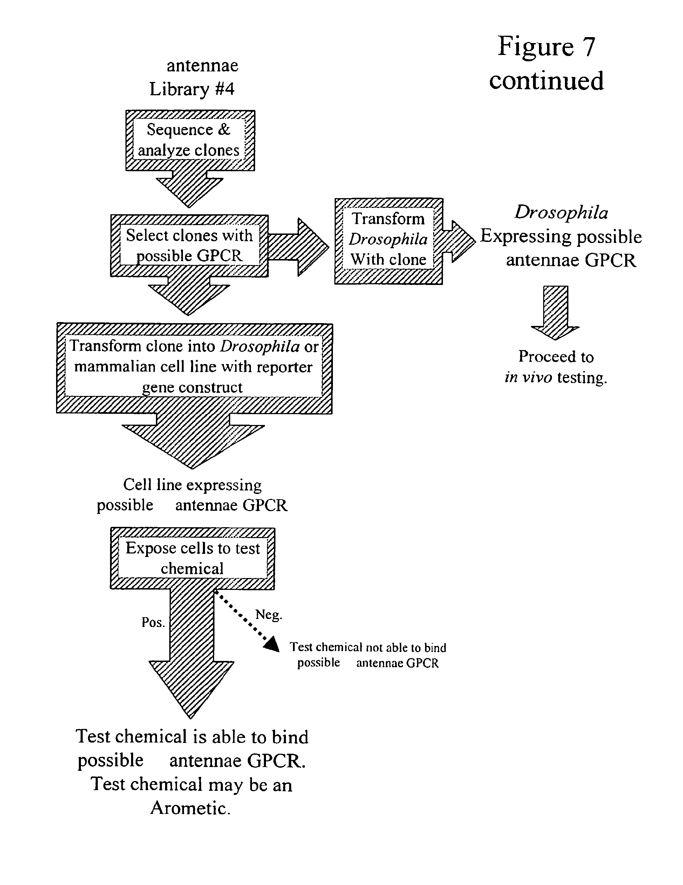

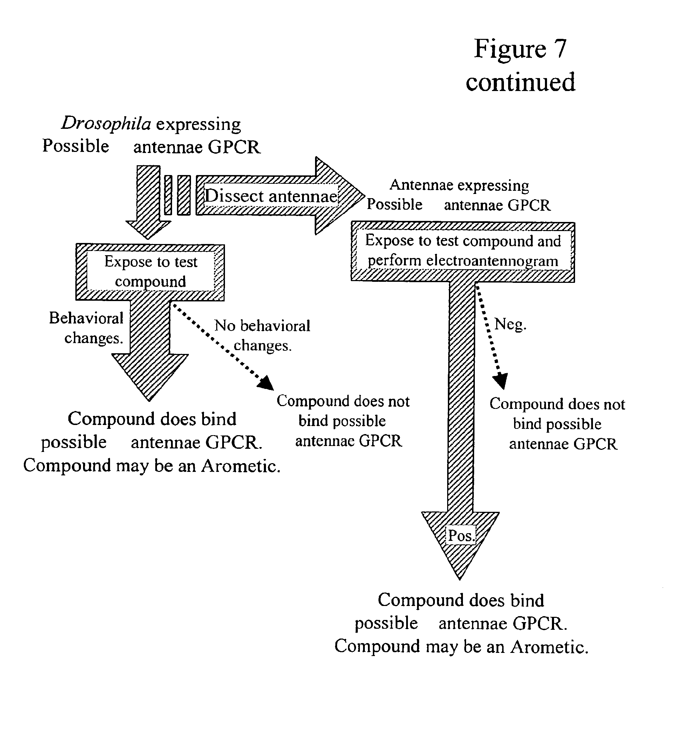

**FIG. 7****.** Flow diagram of the screening processes used to isolate male-specific-tissue-specific genes and proteins and female-specific-tissue-specific genes and proteins and to screen for compounds that interact with these proteins.

### Summary

The present disclosure recognizes the need to rapidly and reliably identify novel members of protein families whose known members do not necessarily exhibit useful degrees of sequence homology; the protein families include but are not limited to Seven-Transmembrane Proteins like the GPCRs. The present disclosure also recognizes the need to identify potential interactors for these proteins. The present disclosure permits the identification of novel GPCRs and their interactors based on differential tissue expression and/or the assay of protein-protein interactions in a cell based system. Since the identification of novel GPCRs and their interactors is performed in living cells, cell lines can be developed to allow further functional characterization and/or the isolation of other interacting proteins or effectors, regardless of whether these effectors or interactors are of synthetic, partially synthetic or natural origin. The present disclosure thus provides distinct advantages over existing methods to isolate, identify, and characterize novel protein family members and their interactors. The present disclosure also permits the rapid identification of OBPs and other olfactory proteins, and potential interactors for these proteins.

Applications of the present disclosure include but are not limited to the development of novel, non-toxic, species-specific pesticide alternatives that are compliant with the Food Quality Protection Act (FQPA) and operate based on mating disruption or the alteration of other scent-controlled behaviors in arthropod, other invertebrate, or vertebrate pests, and the development of pest monitoring systems that operate based on the presence of pest pheromone *in situ.* Furthermore, the present disclosure can be used to isolate a host of novel semiochemicals with desirable effects on specific species.

### DETAILED DESCRIPTION OF THE INVENTION

### Definitions

Unless otherwise stated, all scientific and technical terms used herein have the same meaning as commonly understood by one of ordinary skill in the art to which this invention belongs. Generally, the nomenclature used herein and the laboratory procedures in molecular biology, molecular genetics, biochemistry, physical chemistry, cell culture, protein chemistry, and nucleic acid chemistry described below are those well known and commonly employed in the art. Standard techniques are used for recombinant nucleic acid methods, eukaryotic transformation, and microbial culture and transformation. Enzymatic reactions and purification steps are performed according to the manufacturer's instructions unless otherwise noted. Techniques and procedures are generally performed according to conventional methods in the art. General references include Sambrook et al., Molecular Cloning: A Laboratory Manual, 2nd Ed. (1989) Cold Spring Harbor Laboratory Press, Cold Spring Harbor, N.Y., USA, and Ashbumer, M., Drosophila: A Laboratory Manual (1989) Cold Spring Harbor Laboratory Press, Cold Spring Harbor, N.Y., USA. The laboratory procedures described in combinatorial chemistry, synthetic chemistry, and electrophysiology, and the nomenclature used are those well known and commonly employed in the art. As employed throughout the disclosure, the following terms, unless otherwise indicated, shall be understood to have the following meanings:
"Agonist" refers to a molecule that binds a receptor and causes its activation, leading to a signal being transduced or converted that elicits a certain behavioral response (for example, a pheromone molecule that induces mating behavior).
"Anchored primer" refers to an oligonucleotide physically attached to an object or surface, such as a glass bead.
"Anosmia" refers to the inability to detect an odor or odors.
"Antagonist" refers to a molecule that binds a receptor and blocks its activation by an agonist, (for example, a molecule that inhibits mating behavior).
"Arometics" refers to small synthetic molecules isolated from the combinatorial chemical libraries that will act as either agonists or antagonists to the targeted pheromone receptors. Although they can bind the same receptors as native pheromones, Arometics are not the native pheromone. The term "Pheromone mimetics" is also used to describe these molecules.
"Attractants" refers to compounds or molecules capable of attracting a pest species.
"Bioinformatics" refers to the discipline that integrates biotechnology and modem computational, statistical, and analytical or mathematical methods.
"Bioscensor" refers to an application of the present disclosure that permits the detection of semiochemicals *in situ.*
"cDNA" refers to complementary DNA, which is a DNA copy of the mRNA or messenger RNA expressed in the cell. The term "cDNA" therefore represents gene products or transcripts.
"Codlemone" refers to pheromone of the codling moth, *Cydia pomonella Linnaeus.*
"Combinatorial chemical libraries" refers to large, optionally randomly constructed libraries of small molecules; these libraries are used in screening for potential substitutes to naturally occurring pheromones.
"Differential Gene Expression" or "differential expression" refers to genes that are expressed selectively in one subpopulation of a species but not another, or in one area of the body but not another. For example, the term may be used to refer to genes expressed by one gender of an insect species but not another, or in a specific body part (for example, the antennae) but not other parts of the body.
"DiI" refers to a hydrophobic fluorescent dye specifically incorporated into cell membranes used in visualization experiments and processes.
"Domain" refers to an area of a protein with a specific function or exhibiting a specific structural motif.
"Effectors" refers to naturally occurring, synthetic or partially synthetic molecules, or compounds capable of interacting with a receptor under study. Effectors can be agonists or antagonists.
"Electroantennogram" refers to the output of a device incorporating electrodes that measure electrical activity across an antenna mounted in conductive medium, such as a gel. In this manner, the response of receptors on the antenna to stimuli including odors can be quantified.
"Female Library" refers to a cDNA representing genes expressed selectively in females of a species under scrutiny. Such a probe can be tissue-specific, representing the sum of gene products expressed in a selected tissue of females.
"Female Probe" refers to a probe representing the sum of the gene products expressed selectively in females of a species under scrutiny.
"FRET" refers to Fluorescent Resonance Energy Transfer, an energy transfer phenomenon between two bodies in close proximity that is used in biological microscopy to assay interactions.
"FQPA" refers to the Food Quality Protection Act of 1996 that requires all present tolerances for pesticides to undergo risk assessments under more stringent standards; the implications of this legislation suggest that several widely used organophosphates and carbamates will be slowly phased out over a ten-year period.
"Functional biology" refers to assaying the function of the isolated GPCRs utilizing a biological activity that can be measured in the laboratory.
"Genomics" refers to the cloning and molecular characterization of entire genomes.
"Genetically Modified Organism (GMO)" refers to an organism that has been genetically engineered using gene splicing or molecular biology techniques (vs. a traditional breeding approach) to exhibit specific genetic traits.
"G-protein coupled receptors (GPCRs)" refers to pheromone or odorant receptor molecules that bind trimeric G-proteins within the cell.
"High throughput bioassay" refers to an assay system based on a biological response that can be accomplished on a very large scale (>1000/day).
"High throughput sequencing" refers to a DNA sequencing system that allows for the determination of a very large number of nucleotides (>32,000 base pairs/day).
"Homologs" refers to genes that have a common ancestry. Homologs are divided into orthologs, which are homologs with the same function as the ancestral gene, and paralogs, that are homologs with a different function from the ancestral gene.
"Homology" refers to the extent of similarity between the DNA sequences encoding two or more genes, or the amino acid sequences comprising two or more proteins, as in a gene or protein family.
"Hybridization" refers to selective and specific binding, typically between a probe and its target.
"Hydrophobicity" refers to the solubility of a particular entity such as a protein or chemical in water.
"Known pheromone" refers to a pheromone already identified that mediates a specific behavioral response.
"Lead Chemical" refers to a chemical suspected to have the ability to interact with a GPCR, thus making it a candidate pheromone mimetic or arometic.
"Male Library" refers to a cDNA library representing genes expressed selectively in males of a species under scrutiny. Such a probe can be tissue-specific, representing the sum of gene products expressed in a selected tissue of males.
"Male Probe" refers to a probe representing the sum of the gene products expressed selectively in males of a species under scrutiny.
"Mating disruption" refers to a method of pest control most commonly found in agriculture; it involves saturating the crop environment with a sex pheromone in order to confuse the males and prevent them from locating females.
"mRNA" refers to that portion of RNA comprising sequences that are translated into proteins. Only a portion of the RNA present in a cell is mRNA; other RNA forms include ribosomal RNA and transfer RNA.
"Odorant Binding Proteins" or "OBPs" refers to proteins in sensory tissues believed to bind odors, that are typically hydrophobic, and escort them across the hydrophilic extracellular matrix to the cell surface, where odorant receptors are located.
"Odorant Receptor" refers to the subcellular structures located in the plasma membrane of insect neuronal cells that are responsible for initiating the organism's perception of a specific odor - that is, they allow the organism to smell various scents and odors.
"Odorant" refers to smell, scent, or odor.
"PCR" refers to the Polymerase Chain Reaction, a method of amplifying nucleic acid sequences *in vitro* in order to obtain larger amounts of DNA.
"Pheromone Bioscensor" refers to a cell-based pheromone detection device.
"Pheromone Mimetics" refers to small synthetic molecules isolated from the combinatorial chemical libraries that will act as either agonists or antagonists to the targeted pheromone receptors. Although they can bind the same receptors as native pheromones, Pheromone Mimetics are not the native pheromone. The term "Arometics" is also used to describe these molecules.
"Phastscreen" refers to one or more *Drosophila* cell lines that have been genetically engineered to express functional GPCRs with activity that can be assayed.
"Pheromone receptors" refers to GPCRs that bind the specific pheromone molecule.
"Pheromone" refers to an odorant chemical released by an insect that causes a specific interaction with another insect of the same species.
"Phretcheck" refers to a *Drosophila,* mammalian, or other transformed eukaryotic cell line that can be used to directly isolate GPCRs based on the receptor's interaction with a G-protein.
"Probe" refers to a labeled DNA fragment, RNA fragment, protein fragment, or chemical that can hybridize to a specific region of a target DNA or protein segment, and whose presence can be readily assayed.
"Promotor" refers to a segment of DNA that controls gene expression *in vivo,* capable of limiting expression spatially and/or temporally.
"Reagents" refers to chemicals and compounds (either naturally occurring, synthetic or partially synthetic) or enzymes used in a chemical reaction to measure or yield other substances.
"Repellents" refers to compounds or molecules capable of repelling a pest species.
"Reporter Gene" refers to a gene used in biological or biochemical experiments in order to monitor an interaction. Reported genes respond to protein-protein interactions by triggering an effect that is easily detectable, for example, the emission of fluorescent light or the production of an assayable product.
"Semiochemicals" refers to chemicals (such as, pheromones or pheromone-like compounds) that mediate interactions between organisms.
"Serpentine receptors" refers to GPCRs; this term is based on the actual structure of the protein in the cell membrane (seven transmembrane passes in a serpentine shape).
"Signal transduction cascade" refers to a series of molecules in a cell that transduces or converts an external signal (for example, a pheromone) into a downstream response within the cell (for example, a change in gene activity).
"Trans-gene" refers to a gene that has been introduced into the genome of a cell or organism by transformation.
"Transmembrane Domains" refers to hydrophobic domains of a protein that penetrate the cell membrane.
"Unknown pheromone" refers to a pheromone not yet determined or identified that mediates a specific behavioral response.

### Introduction

The scope of the invention is defined in Claims. More particularly, the present invention concerns a method of reducing a target animal's sensitivity to odors, comprising;
a) providing a compound known to interact with OBPs of a target animal ;
b) incorporating said compound into products capable of altering said target animal behavior; and
c) exposing said target animal to the product containing said compound.

The present disclosure recognizes the need to identify novel members of protein families whose known members do not necessarily exhibit high or useful degrees of sequence homology; such families include but are not limited to Seven-Transmembrane Proteins or Serpentine receptors like the GPCRs, and odorant binding proteins or OBPs. The present disclosure provides the means and constructs to rapidly array and screen cDNA libraries for such proteins, and recognizes the need to identify potential interactors for these proteins and assay their activity. Thus, the present disclosure permits the identification of novel odorant proteins and their interactors based on differential tissue expression and/or the assay of protein-protein interactions in either a cell based or non-cell based system. Since the identification of novel odorants and their interactors can be performed in living cells, cell lines can be developed to allow further functional characterization and/or the isolation of other interacting proteins or effectors, regardless of effector origin (synthetic or naturally occurring substance). The present disclosure thus provides distinct advantages over existing methods used to isolate, identify, and characterize novel protein family members and their interactors.

What follows is a non-limiting introduction to the breadth of the present disclosure, including several general and useful aspects:
1) The present disclosure provides a method for identifying pheromone receptors specifically or GPCRs in general from a species with no known receptors. The identification can be achieved without relying on sequence homology to known members of a protein family and without a known agonist, antagonist, or other effector for the GPCR or receptor. Identification of pheromone receptors instead relies on comparing genes expressed in the sensory organs (antennae) of male and female organisms, then isolating differentially expressed transcripts. The DNA sequence of these transcripts is then analyzed for the presence of transmembrane domains in order to segregate only those transcripts that have the seven-transmembrane-domain structure characteristic of pheromone receptors.
2) The present disclosure provides a method, called "Phretcheck," to rapidly identify genes encoding proteins that interact with Gα, a subunit of a trimeric G-protein known to interact with GPCRs. Therefore, the present disclosure provides another means of identifying potential GPCRs. Other Serpentine receptors or desirable proteins, including OBPs, can be isolated in a similar manner.
3) The present disclosure provides a method for identifying molecules, chemicals, or reagents, either synthetic, occurring in nature or partially synthetic, that interact with isolated odorant receptors without prerequisite knowledge of the native ligand's structure. Identification is accomplished using a cell-based system that allows high-throughput assays. This method can therefore employ large combinatorial chemistry libraries to identify receptor ligands, whether the ligands are agonists or antagonists. These ligands may mimic the function of native pheromones, and are therefore called "Arometics" or "Pheromone Mimetics."
4) The present disclosure provides a method to assay the activity of potential pheromone mimetics or Arometics using transformed *Drosophila melanogaster,* either as a transformed whole organism, transformed dissected sensory organs, or cultured transformed cell lines. For example, a novel GPCR can be transformed into *Drosophila* using tools readily available in the art, and then assayed in any of three different manners for interaction with a lead compound identified from assaying a combinatorial chemical library as described herein:
   (a) The entire transformed organism can be exposed to the lead chemical and assayed for a behavioral response.
   (b) The antennae from transformed *Drosophila* can be dissected and assayed for a response to the lead chemical using an electroantennogram or similar method.
   (c) The transformed organisms can be used to develop stable cell lines that can be cultured *in vitro,* and these cell lines can be assayed for a response to the lead chemical via a variety of methods, including but not limited to coupling the GPCR to a reporter gene cascade.

The present disclosure provides a method to assay the concentration or presence of natural, synthetic or partially synthetic semiochemicals present in a given area; furthermore, the concentration or presence of any effector capable of binding a known GPCR can be determined *in situ.* Such a device, called a "Bioscensor," is capable of indicating whether a specific area is infected with a pest based on detecting the pheromones emitted into the atmosphere by that pest. The device is also capable of detecting pheromones applied as a means of pest control.

The present disclosure provides a method to rapidly array a cDNA library while normalizing the frequency of clone representation. This method allows complex cDNA libraries to be efficiently analyzed; the contents of the library are normalized to prevent rare transcripts from being under-represented during analysis.

The technologies, methods, and compositions presented herein can be adapted to isolate other cDNA clones, and by extension other proteins, by rapidly screening cDNA libraries generated from tissues or organisms of interest.

The present disclosure provides methods, technologies, and compositions desirable to induce anosmia in a variety of species, ranging from arthropods to humans. Applications range from pest control to odor masking in agricultural, commercial, and domestic environments. The species this aspect of the present disclosure can be applied to include but are not limited to the following and include non-human animals, non-human vertebrates, non-human mammals and non-human primates.

Invertebrates: isoptera (for example, termites), hemiptera (for example, sharpshooters of the genus *Homalodisca*), heteroptera (for example, kissing bugs of the genus *Triatoma*), homoptera (for example, whiteflies, aphids, scales), lepidoptera (for example, moths, butterflies), hymenoptera (for example, wasps, ants, sawflies, bees, hornets), coleoptera (for example, beetles), diptera (for example, mosquitoes, gnats, flies), orthoptera (for example, grasshoppers and locusts), blattaria (for example, cockroaches), mantodea (for example, mantices), thysanura (for example, silverfish, firebrats), psocoptera (for example, booklice), siphonaptera (for example, fleas) phthiraptera (for example, lice), myriapods (for example, millipedes and centipedes), other insects, mites, spiders, ticks, other arachnids, terrestrial isopods (for example, pill bugs and sow bugs), other arthropods, annelids, nematodes, mollusks (for example, snails and slugs).

Vertebrates: rodents, lagomorphs (for example, rabbits), insectivora (for example, moles and shrews), chiroptera, carnivora (for example, weasels, coyotes, bears, dogs, and cats), artiodactyls, perissodactyls, primates (including humans), other mammals, reptiles, marine vertebrates including agnatha, chondrichthyes (for example, sharks) and osteichthyes, aves (for example, pigeons, starlings).

### I. Methods for Identifying Novel G-Protein Coupled Receptors or Odorant-Binding Proteins.

One aspect of the present disclosure is a method for identifying nucleic acid molecules encoding novel sex-linked-tissue-linked receptors, comprising: providing a male-specific-tissue-specific nucleic acid molecule library from a tissue of interest isolated from males of an animal of interest and providing a female-specific-tissue specific nucleic acid molecule library from the tissue of interest isolated from females of the animal of interest. This aspect of the present disclosure further comprises sequencing the inserts of the male-specific and female-specific tissue-specific nucleic acid molecules, followed by analyzing the sequences of the inserts, such that nucleic acid molecules encoding possible male-specific-tissue-specific proteins and female-specific-tissue specific proteins are identified. In general, the nucleic acid molecule libraries are derived from expressed nucleic acid molecules.

Another aspect of the present disclosure is that the animal of interest may be an invertebrate. Invertebrate animals can include, but are not limited to dipterans, mosquitoes, gnats, flies, termites, lepidopterans, moths, butterflies, orthopterans, grasshoppers, locusts, sharpshooters, *Homalodisca spp.,* cockroaches, beetles, ants, fleas, silverfish, booklice, firebrants, hymenopterans, wasps, bees, hornets, kissing bugs, *Triatoma dimidiatamyria,* other insects, myriapods, millipedes and centipedes, mites, spiders, ticks, other arachnids, terrestrial isopods, pill bugs and sow bugs, other arthropods, annelids, nematodes, mollusks, snails and slugs.

In a further aspect of the present disclosure, the animal of interest may be a vertebrate. Vertebrate animals include, but are not limited to rodents, lagomorphs, insectivora, moles and shrews, chiroptera, carnivora, weasels, coyotes, bears, dogs and cats, artiodactyls, perissodactyls, primates, humans, other mammals, reptiles, marine vertebrates including agnatha, chondrichthyes, sharks, osteichthyes, aves, pigeons. In yet another aspect of the present disclosure the tissue of interest is derived from a sensory organ, such as an olfactory organ, nose or antennae.

An additional aspect of the present disclosure is that the insert sequences may be analyzed by bioinformatic techniques. For example, sequences of particular interest may contain at least one hydrophobic transmembrane domain motif, such as those encoded by a seven-transmembrane receptor. Preferably, the sequence encodes a GPCR, more preferably the GPCR is an odorant receptor.

Yet another aspect of the present disclosure is an isolated nucleic acid molecule encoding the GPCR. Preferably the isolated nucleic acid molecule is an expression vector encoding the GPCR operably linked to a promoter. A preferable aspect of the present disclosure is an isolated nucleic acid molecule encoding an odorant receptor. Still more preferable, the isolated nucleic acid molecule is an expression vector encoding the odorant receptor operably linked to a promoter.

The present disclosure provides a method for isolating receptors or odorant binding proteins by exploiting differential gene expression in the sensory organs or antennae of male and female animals, such as insects. Animals are collected and the two genders segregated. Antennae are dissected from each gender and frozen on dry ice; the bodies of each gender are also frozen. The tissue samples are then separately used to isolate mRNA, which is transcribed *in vitro* and used to construct cDNA libraries in a suitable vector. These cDNA libraries represent the genes expressed in the sensory organs of males and females, and in the bodies of males and females. The libraries can be arrayed and screened for transcripts that are specifically expressed in the antennae of either gender. This is accomplished as follows:
Arrayed clones are plated on surfaces suitable for hybridization, such as nylon, nitrocellulose or silica, and screened with probes made from mRNA that is isolated from bodies lacking antennae. Positive clones are discarded in order to select for clones representing RNA expressed specifically in the antennae. The pooled female antennal cDNA and the pooled male antennal cDNA are used separately to make two probes, one representing all RNA expressed in male antennae and one representing all RNA expressed in female antennae. Next, the male library is screened with the female probe to isolate male-specific clones (those clones that cannot hybridize to the female probe). The male library is screened with the male probe to eliminate vectors lacking an insert. The female library is screened with the male probe to isolate female-specific clones (those clones that do not tend to or cannot hybridize to the male probe). The female library is screened with the female probe to eliminate or reduce vectors lacking an insert. Clones that meet the following criteria are isolated:
   Clones that are not represented in libraries derived from the bodies of either gender; and clones that are represented in the libraries of the gender from which they are derived, and clones that are not represented in the library of the gender from which they are not derived.

This screening process results in the isolation of tissue-specific and gender-specific clones; all clones isolated tend to or are present only in one gender and tend to or only in one tissue type, namely, the sensory, such as antennal, tissues where odorant GPCRs or OBPs are normally expressed. The isolated clones are optionally sequenced; a high throughput sequencing approach may be desirable depending on the number of clones isolated. One or more clones encoding potential GPCRs are then optionally identified based on bioinformatic analysis available in the art, which can identify the probable presence of motifs or domains characteristic of GPCRs. These motifs or domains are structural features like the seven hydrophobic transmembrane domains that are characteristic of GPCRs. Clones encoding potential OBPs can be identified based on similar principles. In this manner, the present disclosure provides a means of identifying novel GPCRs or OBPs that does not depend on amino acid sequence homology.

As an alternative to isolating gender-specific clones, the libraries with clones from the sensory tissue, such as antennae, of both genders are arrayed on a suitable support, such as nitrocellulose, nylon or silica; and screened with a labeled probe made from cDNA expressed in the bodies. Positive clones are discarded in order to enrich for those clones specific to sensory tissue, since the genes encoding OBPs will tend to be expressed selectively in sensory tissues rather than elsewhere in the body. The female sensory tissue library is screened with a probe derived from female sensory tissue cDNA and negative clones are eliminated or reduced because they lack an insert. Optionally, highly expressed clones are sequenced and optionally the data analyzed for the presence of cDNAs encoding OBPs. Approximately 15% of all antennal cDNAs have been found to encode OBPs.

### II. Methods for Rapidly Detecting Proteins that Interact with Gα Proteins, "Phretcheck"

Another aspect of the present disclosure is a method of rapidly detecting proteins that interact with species-specific Gα proteins comprising: providing cell clones lacking both the endogenous Gα protein and the Gα protein's associated receptor and expressing an animal-specific-tissue-specific Gα protein gene fused to a reporter gene and each of said cell clones expressing the nucleic acid molecule of a potential Gα protein-interacting protein; providing a library of test compounds; contacting the clones with one or more test compounds; detecting a signal from clones having an interaction between the expressed tissue-specific Gα protein and expressed potential Gα protein-interacting protein; isolating the expression vector from the positive clones; and optionally sequencing the nucleic acid molecule of the Gα protein-interacting protein, whereby the protein capable of interacting with the tissue-specific Gα protein is identified. Preferably, the reporter gene expresses a protein assayable by FRET, such as BFP. Preferably, the Gα protein-interacting protein is a GPCR, especially an odorant receptor.

Still another aspect of the present disclosure is that the animal of interest may be an invertebrate. Invertebrate animals can include, but are not limited to dipterans, mosquitoes, gnats, flies, termites, lepidopterans, moths, butterflies, orthopterans, grasshoppers, locusts, sharpshooters, *Homalodisca spp.,* cockroaches, beetles, ants, fleas, silverfish, booklice, firebrants, hymenopterans, wasps, bees, hornets, kissing bugs *Triatoma dimidiatamyria,* other insects, myriapods, millipedes and centipedes, mites, spiders, ticks, other arachnids, terrestrial isopods, pill bugs and sow bugs, other arthropods, annelids, nematodes, mollusks, snails and slugs.

In a further aspect of the present disclosure, the animal of interest may be a vertebrate. Vertebrate animals include, but are not limited to rodents, lagomorphs, insectivora, moles and shrews, chiroptera, carnivora, weasels, coyotes, bears, dogs and cats, artiodactyls, perissodactyls, primates, humans, other mammals, reptiles, marine vertebrates including agnatha, chondrichthyes, sharks, osteichthyes, aves, pigeons.

In yet another aspect of the present disclosure, the tissue of interest is derived from a sensory organ, such as an olfactory organ, nose or antennae.

An additional aspect of the present disclosure is that the insert sequences may be analyzed by bioinformatic techniques. For example, sequences of particular interest may contain at least one hydrophobic transmembrane domain motif, such as those encoded by a seven-transmembrane receptor. Preferably, the sequence encodes a GPCR, more preferably the GPCR is an odorant receptor.

Yet another aspect of the present disclosure is an isolated nucleic acid molecule encoding the GPCR. Preferably the isolated nucleic acid molecule is an expression vector encoding the GPCR operably linked to a promoter. A preferable aspect of the present disclosure is an isolated nucleic acid molecule encoding an odorant receptor. Still more preferable, the isolated nucleic acid molecule is an expression vector encoding the odorant receptor operably linked to a promoter.

The present disclosure provides a method, involving a cell-based assay, for the rapid detection of proteins that interact with Gα protein. Since Gα proteins are subunits of the heterotrimeric protein complex directly associated with G-Protein Coupled Receptors, the present disclosure provides a method to detect GPCRs based on protein-protein interactions. This method can utilize any established cell line *in vitro.* For example, yeast cell strains, mammalian cell strains, or insect cell strains can be used.

Cell strains lacking the endogenous Gα protein and associated receptor are preferred to minimize possible interference of the endogenous protein and receptor with the present disclosure's ability to detect interactions between transformed Gα protein and receptors. What follows is a general example of the present disclosure:
Hybridization screens based on PCR and degenerate probes can be used to isolate cDNAs encoding Gα proteins, which are typically highly conserved across species. Once a particular Gα protein clone has been isolated, a short C-terminal sequence from the Gα protein expressed in the antennae of a target insect species fused to a reporter protein known or later developed (GFP or Green Fluorescent Protein, for example) can be transformed into established cell lines. Such a construct, if targeted correctly by the cell, will tend to be found predominantly in the cytoplasm. This cell strain will then be transformed with cDNA libraries constructed from transcripts expressed in the antennal tissue of the specific target species. The C-terminus of the Gα product interacts with GPCRs *in vivo*, and this interaction can be assayed *in vitro* using the reporter gene - Gα protein construct already transformed into the cells provided the cells also incorporate a fluorescent membrane label: After transformation, if a cultured cell expresses a GPCR, the Gα-GFP fusion construct should interact with it and result in a translocation of the fusion construct from the cytoplasm to the cell membrane. This event will in turn result in FRET emission as the Gα protein and the fluorescently labeled cell membrane come into proximity. This FRET emission is readily detectable under fluorescent microscopy, flow cytometry, or other means including but not limited to optical analysis. Cells fitting the screen criteria will be run through the analysis a second time for confirmation. Upon confirmation, the transformed fusion construct consisting of the candidate GPCR and fluorescent reported gene will be isolated and optionally sequenced to determine the identity of the candidate GPCR

### III. Methods to Rapidly Identify Compounds that Bind Receptors Located on or Within the Plasma Membrane in vitro: "Phastscreen"

Yet another aspect of the present disclosure is a method of identifying compounds that bind receptors located on or within a plasma membrane comprising: providing at least one cell expressing a Gαo hybrid protein functionally linked to an assayable ion channel that allows influx of calcium into the cell, and a target protein; providing a library of test compounds; contacting said cell with at least one test compound; and detecting a calcium influx, whereby a compound capable of interacting with the target protein is identified. Preferably, the calcium influx is detected with a protein that emits light when the protein binds free calcium or a fluorescent dye. More preferably, the protein that emits light is Aequorin. In further aspects of the present disclosure, the target protein is a seven-transmembrane protein, such as a GPCR, preferably an odorant receptor. A still further aspect of the present disclosure is a compound identified by the method described herein.

Yet another aspect of the present disclosure is a method of identifying compounds that bind receptors located on or within the plasma membrane comprising: providing at least one cell expressing a BFP/Gα hybrid protein functionally linked to a YFP/Gβ hybrid protein, and a target protein; providing a library of test compounds; contacting said cell with a test compound; and detecting FRET, whereby a compound capable of interacting with the target protein is identified.

In preferred aspects of the present disclosure, the GPCR is an odorant receptor.

Another preferred aspect of the present disclosure is a compound identified by the method described herein.

The present disclosure provides novel methods to combine the power of genetics with the ease of *in vitro* culture to rapidly identify compounds that will bind to receptors that are located on or within the plasma membrane *in vitro.* These methods can be used for high-throughput screening.

In the following example, *Drosophila melanogaster* is used, but any other insects or animals can be used. An insect intermediate can be advantageous if the receptors being studied are derived from insects, since correct, successful expression of transformed receptors is most likely in similar hosts.

### IV. Methods to Identify Small Synthetic Molecules that are GPCR or OBP Agonists or Antagonists: "Arometics"

In certain aspects of the present disclosure, the animal is an invertebrate. In general, the invertebrate is selected from the group consisting of dipterans, mosquitoes, gnats, flies, termites, lepidopterans, moths, butterflies, orthopterans, grasshoppers, locusts, sharpshooters, *Homalodisca spp.*,cockroaches, beetles, ants, fleas, silverfish, booklice, firebrants, hymenopterans, wasps, bees, hornets, kissing bugs, *Triatoma dimidiatamyria,* other insects, myriapods, millipedes and centipedes, mites, spiders, ticks, other arachnids, terrestrial isopods, pill bugs and sow bugs, other arthropods, annelids, nematodes, mollusks, snails and slugs.

In other aspects of the present disclosure, the animal is a vertebrate. Preferably, the vertebrate is selected from the group consisting of rodents, lagomorphs, insectivora, moles and shrews, chiroptera, carnivora, weasels, coyotes, bears, dogs and cats, artiodactyls, perissodactyls, primates, humans, other mammals, reptiles, marine vertebrates including agnatha, chondrichthyes, sharks, osteichthyes, aves, pigeons.

A preferred aspect of the present disclosure is a compound identified by the methods described herein. In more preferred aspects of the present disclosure, the compound identified is an Arometic.

The present disclosure provides a method to identify small molecules that mimic the effect of natural odors or scents, including natural pheromones and odors used by any pest species, animal or insect, in particular to identify their potential mates, food sources, or other aspects of their environment. "Arometics" are small synthetic molecules isolated from combinatorial chemical libraries that will act as either agonists or antagonists to the targeted pheromone receptors. Although they can bind the same receptors as native pheromones, Arometics are not the native pheromone.

To develop Arometics, the GPCR or OBP controlling a specific behavior such as mating or feeding is identified using one of the methods already provided by this disclosure. Once this protein has been isolated, it can be incorporated into a reporting system including transgenic eukaryotic cell lines (Phastscreen, described herein). Combinatorial chemical libraries are screened for compounds capable of interacting with the GPCR or OBP *in vitro* or *in vivo,* and these compounds are then incorporated into products capable of altering pest species behavior based on their scent.

### V. Methods to Determine the Presence of Natural or Synthetic Semiochemicals in situ: "Bioscensor"

Another aspect of the present disclosure is a method of determining the presence of natural or synthetic semiochemicals *in situ,* comprising: providing a GPCR capable of recognizing said semiochemical of interest; incorporating said GPCR into a reported cascade; and detecting activation of said reporter cascade, whereby the presence of said semiochemical is detected. In preferred aspects of the present disclosure, the reporter cascade causes a color change or an electrical change that can be measured. In certain aspects of the present disclosure, activation of the reporter cascade is detected enzymatically, chemically or electrically. In preferred aspects of the present disclosure, the GPCR is an odorant receptor.

The present disclosure provides a novel method to determine whether a given semiochemical is present in a liquid or gaseous solution, including the atmosphere, provided the protein (a GPCR and/or OBP) capable of recognizing the semiochemical in question is known. This protein can be isolated using one of the methods described herein, and incorporated into an enzymatic, chemical, or electrical reporter cascade that produces a visible product such as a color change on an indicator strip or a measurable change in electrical properties of a silica surface.

### VI. Methods to Develop Devices that Reduce a Target Species' Sensitivity to Odors

Yet another aspect of the present invention is a method of reducing a target animal's sensitivity to odors, comprising; providing a compound known to interact with OBPs of a target animal; incorporating said compound into products capable of altering target animal behavior; and exposing said target animal to the product containing said compound.

In preferred aspects of the present invention the animal is an invertebrate. More preferably, the invertebrate is selected from the group consisting of dipterans, mosquitoes, gnats, flies, termites, lepidopterans, moths, butterflies, orthopterans, grasshoppers, locusts, sharpshooters, *Homalodisca* spp.,cockroaches, beetles, ants, fleas, silverfish, booklice, firebrants, hymenopterans, wasps, bees, hornets, kissing bugs, *Triatoma dimidiatamyria,* other insects, myriapods, millipedes and centipedes, mites, spiders, ticks, other arachnids, terrestrial isopods, pill bugs and sow bugs, other arthropods, annelids, nematodes, mollusks, snails and slugs.

In other preferred aspects of the present disclosure the animal is a vertebrate. More preferably, the vertebrate is selected from the group consisting of rodents, lagomorphs, insectivora, moles and shrews, chiroptera, carnivora, weasels, coyotes, bears, dogs and cats, artiodactyls, perissodactyls, primates, other mammals, reptiles, marine vertebrates including agnatha, chondrichthyes, sharks, osteichthyes, aves and pigeons.

Animals, such as insects, including mosquitoes, such as *Anopheles gambiae,* use olfactory stimuli as a means of identifying potential blood meal hosts. Consequently, devices that reduce the animal's, such as mosquitoes,' sensitivity to odors can control pests in an environmentally responsible manner. the present invention recognizes this need and provides means, methods, and constructs to develop devices capable of reducing animal, such as mosquito, sensitivity to the odors commonly used to locate hosts, including humans and non-humans. The goal of these devices is to induce anosmia in as many species of animals, such as mosquito, as possible. Since OBPs are well conserved across species, such a goal is realistic.

OBPs from several target species can be isolated as provided by this disclosure and described herein. For example, if the target species is an insect, OBPs can be isolated as follows:
Animals are collected; sensory organs, such as antennae, are dissected and frozen on dry ice. The tissue samples are then used to isolate mRNA, which is transcribed *in vitro* and used to construct cDNA libraries in a suitable vector. These cDNA libraries represent the genes expressed in the sensory organs. The libraries can be arrayed and screened for transcripts that are specifically expressed in the sensory organ, such as antennae. This is accomplished as follows:
   Arrayed clones are plated on surfaces suitable for hybridization, such as nitrocellulose, nylon or silica, and screened with probes made from the antennal cDNA, and the clones that give strong hybridization signals are picked and sequenced.

Clones that are highly expressed in the sensory organs, such as antennae, can be isolated using the methods described here. For example, screening female *Anopheles* library with a probe representing the cDNA pool the library we derived from gives a series of strong signals, many of which are OBPs. These OBPs represent as much as 15% of the total gene transcripts in the antennae of *Anopheles* mosquitoes and are not expressed in a gender-specific manner; they are nonetheless highly expressed and can be isolated.

Once cloned, DNA sequences encoding these OBPs can be inserted into expression vectors so that OBPs can be expressed *in vitro,* using tools common in the art that include transgenic eukaryotic cell lines. Combinatorial chemical libraries are screened for compounds capable of interacting with the OBPs *in vitro* or *in vivo,* and these compounds are then incorporated into products capable of altering pest species behavior based on their scent.

### VII. Methods to Develop Compounds or Constructs that Mask Odors

An additional aspect of the present disclosure is a method of manufacturing compounds or devices that mask odors, comprising: providing human OBP blockers; incorporating said human OBP blockers into a solid, particulate suspension or aqueous gel; and adding said solid, particulate suspension or aqueous gel to a compound or device, such as deodorants, trash cans, fertilizers, cleaning compounds, sprays to neutralize cigarette, cigar and pipe smoke. The present disclosure recognizes the need to develop compounds, devices, or constructs capable of masking undesirable odors in industrial, agricultural, and domestic environments. The present disclosure therefore provides methods and means to incorporate OBP-based or GPCR-based odor-masking technologies into a variety of commercial products or devices.

The present disclosure provides methods for isolating olfactory proteins, including OBPs and GPCRs, by exploiting differential gene expression in the sensory organs of any species. The methods and means provided by the present disclosure can be applied to the isolation of olfactory proteins from humans; nasal tissue from humans can be used to generate tissue-specific human cDNA libraries. These cDNA libraries can be arrayed and screened as follows to isolate OBPs or GPCRs expressed in human nasal tissue:
Nasal tissue is obtained from each gender and frozen on dry ice; tissue samples from the bodies of each gender are also frozen. The tissue samples are then separately used to isolate mRNA, which is transcribed *in vitro* and used to construct cDNA libraries in a suitable vector. These cDNA libraries represent the genes expressed in the olfactory organs of males and females, and in the bodies of males and females. The libraries can be arrayed and screened for transcripts that are specifically expressed in the antennae of either gender. This is accomplished as follows:
   Arrayed clones are plated on surfaces suitable for hybridization, such as nitrocellulose, nylon or silica, and screened with probes made from mRNA that is isolated from bodies lacking nasal tissue. Positive clones are discarded in order to select for clones representing RNA expressed specifically in nasal tissue. The pooled female nasal tissue cDNA and the pooled male nasal tissue cDNA are used separately to make two probes, one representing all RNA expressed in male nasal tissue and one representing all RNA expressed in female nasal tissue. Next, the male library is screened with the female probe to isolate male-specific clones (those clones that do not tend to or cannot hybridize to the female probe). The male library is screened with the male probe to eliminate vectors lacking an insert. The female library is screened with the male probe to isolate female-specific clones (those clones that do not tend to or cannot hybridize to the male probe). The female library is screened with the female probe to eliminate vectors lacking an insert. All clones that meet the following criteria are isolated:
      Clones that are not represented in libraries derived from the bodies of either gender; and clones that are represented in the libraries of the gender from which they are derived; and clones that are not represented in the library of the gender from which they are not derived.

This screening process results in the isolation of tissue-specific and gender-specific clones; all clones isolated tend to or are present only in one gender and or only in one tissue type, namely, the sensory, such as nasal, tissues where odorant GPCRs or OBPs are normally expressed. The isolated clones are optionally sequenced; a high throughput sequencing approach may be desirable depending on the number of clones isolated. One or more clones encoding potential GPCRs are then optionally identified based on bioinformatic analysis available in the art, which can identify the probable presence of motifs or domains characteristic of all GPCRs. These motifs or domains are structural features like the seven hydrophobic transmembrane domains that are characteristic of GPCRs. Clones encoding potential OBPs can be identified based on similar principles. In this manner, the present disclosure provides a means of identifying novel GPCRs or OBPs that does not depend on amino acid sequence homology.

As an alternative to isolating gender-specific clones, clones that are highly expressed in the nasal tissue of either or both genders can be isolated using the methods described here. For example, screening a female human cDNA library with a probe representing the cDNA pool the library was derived from can give a series of strong signals, many of which are OBPs.

DNA sequences encoding these human OBPs or GPCRs can be expressed *in vitro* using tools common in the art. Combinatorial chemical libraries are then screened to isolate compounds capable of interacting with the OBPs or GPCRs. These compounds are selected based on desirable physical, chemical, and biological characteristics - that is, compounds that are non-toxic, relatively simple to manipulate chemically, and capable of strongly binding human OBPs or GPCRs are favored during selection. These compounds are used to block the function of human OBPs or GPCRs by acting as inhibitors, thus lowering the sensitivity of human olfaction. Devices, such as solid deodorizer gels that rely on evaporation for release of the arometic, heating devices that accelerate the evaporation process of the arometic, or electrical devices like atomizers or foggers that emit small amounts of Arometics into the atmosphere, incorporating these OBP-binding or GPCR-binding compounds in a solid form, particulate form in suspension, or aqueous form in a gel have numerous commercial, agricultural, and domestic applications that include but are not limited to the following:
OBP-blocking or GPCR-blocking compounds are incorporated into deodorizing products deployed in public or domestic lavatories found anywhere from airports to homes.
OBP-blocking or GPCR-blocking compounds can be incorporated into deodorizing products used in the home, inside refuse containers. Furthermore, OBP-blocking or GPCR-blocking compounds can be incorporated into the composition of refuse containers themselves to generate odor-resistant containers.
Particulate or liquid OBP-blocking or GPCR-blocking compounds can be introduced in commercially available natural fertilizer to mask the fertilizer's odor in applications requiring an odor-free environment.
OBP-blocking or GPCR-blocking compounds can be incorporated into domestic and industrial cleanser products, and deployed in diverse environments ranging from school cafeterias to slaughterhouses, food processing plants, and restaurants.
Products incorporating OBP-blocking or GPCR-blocking compounds can mask the unpleasant odors associated with cigarette, cigar, and pipe smoke. The compounds can be introduced as an aerosol spray or can be incorporated directly into cigarettes, cigars, or smoking tobacco.

### VIII. Methods to Develop Insect Traps Utilizing Compounds Capable of Attracting a Target Species.

An additional aspect of the present disclosure is a method of trapping invertebrates with odorants, comprising: providing at least one OBP or GPCR agonist; incorporating said agonist into a trap that will selectively attract said invertebrate; and exposing said invertebrate to the trap, whereby said invertebrate is trapped. In further aspects of the present disclosure, the trap further comprises a poison sufficient to kill said trapped invertebrate.
In preferred aspects of the present disclosure, the invertebrate is selected from the group consisting of dipterans, mosquitoes, gnats, flies, termites, lepidopterans, moths, butterflies, orthopterans, grasshoppers, locusts, sharpshooters, *Homalodisca spp*., cockroaches, beetles, ants, fleas, silverfish, booklice, firebrants, hymenopterans, wasps, bees, hornets, kissing bugs, *Triatoma dimidiatamyria,* other insects, myriapods, millipedes and centipedes, mites, spiders, ticks, other arachnids, terrestrial isopods, pill bugs and sow bugs, other arthropods, annelids, nematodes, mollusks, snails and slugs.

Harmful insects, such as mosquitoes such as *Anopheles* and *Culex.* use olfactory stimuli as a means of identifying potential blood meal hosts. Consequently, devices that take advantage of the mosquitoes' sensitivity to odors can control pests by redirecting them away from humans and into traps or lures. The present disclosure recognizes this need and provides means, methods, and constructs to develop devices capable of emitting odors that mimic those odors commonly used to locate human hosts. The goal of these devices is to lure pests so that they can be trapped and optionally subsequently eliminated using conventional insecticides within the trap itself or other means, such as an electrically charged grid.

OBPs or GPCRs from the target species can be isolated as follows: Animals are collected and the two genders segregated. Sensory organs such as antennae are dissected from each gender and optionally frozen on dry ice; the bodies of each gender are also optionally frozen. The tissue samples are then separately used to isolate mRNA, which is transcribed *in vitro* and used to construct cDNA libraries in a suitable vector. These cDNA libraries represent the genes expressed in the sensory organs of males and females, and in the bodies of males and females. The libraries can be arrayed and screened for transcripts that are specifically expressed in the antennae of either gender. This is accomplished as follows:
Arrayed clones are plated on a surface, such as nitrocellulose, nylon or silica, suitable for hybridization and screened with probes made from mRNA that is isolated from bodies lacking antennae. Positive clones are discarded in order to select for clones representing RNA expressed specifically in the antennae. The pooled female antennal cDNA and the pooled male antennal cDNA are used separately to make two probes, one representing some, most or all RNA expressed in male antennae and one representing some, most or all RNA expressed in female antennae. Next, the male library is screened with the female probe to isolate male-specific clones (those clones that do not tend to or cannot hybridize to the female probe). The male library is screened with the male probe to eliminate or reduce vectors lacking an insert. The female library is screened with the male probe to isolate or enrich female-specific clones (those clones that do not tend to or cannot hybridize to the male probe). The female library is screened with the female probe to eliminate or reduce vectors lacking an insert. One or more clones that meet the following criteria are isolated:
   Clones that are not represented in libraries derived from the bodies of either gender, and clones that are represented in the libraries of the gender from which they are derived; clones that are not represented in the library of the gender from which they are not derived.

This screening process results in the isolation of tissue-specific and gender-specific clones; clones isolated tend to be or are present only in one gender and tend to be or are present only in one tissue type, namely, the sensory, such as antennal, tissues where odorant GPCRs or OBPs are normally expressed. The isolated clones are optionally sequenced; a high throughput sequencing approach may be desirable depending on the number of clones isolated. One or more clones encoding potential GPCRs are then optionally identified based on bioinformatic analysis available in the art, which can identify the probable presence of motifs or domains characteristic of all GPCRs. These motifs or domains are structural features like the seven hydrophobic transmembrane domains that are characteristic of all GPCRs. Clones encoding potential OBPs can be identified based on similar principles. In this manner, the present disclosure provides a means of identifying novel GPCRs or OBPs that does not depend on amino acid sequence homology. As an alternative to isolating gender-specific clones, clones that are highly expressed in the antennal tissue of either or both genders can be isolated using the methods described here.

OBPs or GPCRs are then optionally expressed *in vitro,* using tools common in the art that include transgenic eukaryotic cell lines. Combinatorial chemical libraries are screened for compounds capable of interacting with the OBPs or GPCRs *in vitro* and these compounds are then further analyzed to determine which chemical struture(s) yield highly effective OBP or GPCR agonists. Provided the GPCR or OBP targeted controls a behavior that results in the pest being attracted, these compounds will efficiently attract the targeted pest species, and can be incorporated into products capable of altering pest species behavior based on their scent. Such products include but are not limited to traps that selectively attract and kill mosquitoes by incorporating the isolated agonist and a highly toxic pesticide or an electrical grid.

### IX. Methods to Develop Insect Repellents Based on Arometics Acting as Synthetic Agonists

Arometics, described previously herein, can be agonists or antagonists of OBPs or GPCRs. Arometics can be utilized to manipulate any odor-based behavior provided the specific Arometic employed can interact with the GPCR or OBP in the pathway controlling that behavior. If the behavior to be manipulated results in the insect being repelled, Arometics can be used to develop novel insect repellents.

OBPs or GPCRs from the target species can be isolated as follows: Animals are collected and the two genders segregated. Sensory tissues, such as antennae, are dissected from each gender and optionally frozen on dry ice; the bodies of each gender are also optionally frozen. The tissue samples are then separately used to isolate mRNA, which is transcribed *in vitro* and used to construct cDNA libraries in a suitable vector. These cDNA libraries represent at least in part the genes expressed in the sensory organs of males and females, and in the bodies of males and females. The libraries can be arrayed and screened for transcripts that are specifically expressed in the antennae of either gender. This is accomplished as follows:
Arrayed clones are plated on a surface, such as nitrocellulose, nylon or silica, suitable for hybridization and screened with probes made from mRNA that is isolated from bodies lacking antennae. Positive clones are discarded in order to select for clones representing RNA expressed specifically in the antennae. The pooled female antennal cDNA and the pooled male antennal cDNA are used separately to make two probes, one representing some, most or all RNA expressed in male antennae and one representing some, most or all RNA expressed in female antennae. Next, the male library is screened with the female probe to enrich for male-specific clones (those clones that do not tend to or cannot hybridize to the female probe). The male library is screened with the male probe to eliminate or eliminate vectors lacking an insert. The female library is screened with the male probe to isolate or eliminate female-specific clones (those clones that do not tend to or cannot hybridize to the male probe). The female library is screened with the female probe to eliminate or reduce vectors lacking an insert. One or more clones that meet the following criteria are isolated:
   Clones that are not represented in libraries derived from the bodies of either gender; and clones that are represented in the libraries of the gender from which they are derived; and clones that are not represented in the library of the gender from which they are not derived.

This screening process results in the isolation of tissue-specific and gender-specific clones; clones isolated tend to be or are present only in one gender and tend to be or are present only in one tissue type, namely, the antennal tissues where odorant GPCRs or OBPs are normally expressed. The isolated clones are optionally sequenced; a high throughput sequencing approach may be desirable depending on the number of clones isolated. One or more clones encoding potential GPCRs are then optionally identified based on bioinformatic analysis available in the art, which can identify the probable presence of motifs or domains characteristic of all GPCRs. These motifs or domains are structural features like the seven hydrophobic transmembrane domains that are characteristic of all GPCRs. Clones encoding potential OBPs can be identified based on similar principles. In this manner, the present disclosure provides a means of identifying novel GPCRs or OBPs that does not depend on amino acid sequence homology. As an alternative to isolating gender-specific clones, clones that are highly expressed in the antennal tissue of either or both genders can be isolated using the methods described here.

OBPs or GPCRs are then optionally expressed *in vitro,* using tools common in the art that include, but are not limited to, transgenic eukaryotic cell lines. Combinatorial chemical libraries are screened for compounds capable of interacting with the OBPs or GPCRs *in vitro* and these compounds are then further analyzed to determine which chemical struture(s) yield highly effective OBP or GPCR agonists.

### X. Methods to Rapidly Array and Normalize cDNA Clones, Enabling the Efficient Isolation of Specific cDNAs from a Library Regardless of the Frequency of Representation or Library Complexity.

An additional aspect of the present disclosure is a method to rapidly array and normalize nucleic acid molecule clones, enabling efficient isolation of specific nucleic acid molecules from a library regardless of the frequency of representation or library complexity, comprising: functionally linking a common 5' region to all nucleic acid molecules, such that the nucleic acid molecules have a common a site for priming PCR reactions. cDNA libraries derived from specific tissues (for example insect antennae or other organs) and optionally specific genders provide valuable information regarding the genes expressed in those samples. Many libraries can be complex, comprising tens of thousands of clones. During the construction of such libraries, transcript relative abundance may determine the extent to which specific clones are represented in the final product. Gene products expressed at very high levels may be easier to isolate from a cDNA library than gene products expressed at low levels. The present disclosure provides a method to effectively and efficiently screen complex cDNA libraries for relatively rare transcripts by arraying the clones and normalizing the library's content representation.

During library construction, care must be taken in order to ensure the resulting clones will all share a common 5' region useful as a site for priming PCR reactions. This shared 5' region can also include restriction endonuclease recognition sites if so desired. Multi-well plates are used to array such a cDNA library in this method. In each well, oligonucleotides 9between about 1 and about 100, between about 20 and about 80, between about 30 and about 70, preferably about 16) anchored, using established methods, via their 5' ends to a glass bead or other small object of greater mass are present at a nanomolar concentration. These oligonucleotides are designed with a 5' polylinker region containing a host of restriction endonuclease recognition sites, followed by a poly-T region allowing the primer to bind cDNA molecules, and a 3' variable site. **FIG. 5** depicts the oligonucleotides. Although the 5' polylinker and poly-T regions are common among anchored oligonucleotides, each individual oligonucleotide has a unique 3' end. This unique 3' "key sequence" allows an anchored oligonucleotide in each well to selectively hybridize only to those cDNA clones with a complementary 5' end. The "key sequences" are designed to give a comprehensive level of degeneracy since they are diverse and numerous enough to ensure that every possible cDNA sequence can be bound by an individual, specific oligonucleotide in a single well.

Clones from the cDNA library to be screened are heated to denature them into single-stranded DNA and annealed to the anchored oligonucleotides in the wells of the array plates. Since annealing stability depends on the melting temperature of residue bonds in double-stranded DNA, and melting temperatures vary according to the G/C content of specific DNA clones, the annealing reactions are carried out at a variety of temperatures. After annealing, a series of washes are performed to rid the wells of unbound cDNAs, leaving only the annealed duplexes consisting of the anchored oligonucleotides and any specifically bound cDNA clone behind in each well.

PCR reactions are performed using the anchored oligonucleotides as 3' primers and a 5' primer derived from the shared 5' sequence introduced when the cDNA library was constructed. These PCR reactions can be performed directly in the wells of the array plates themselves. The supernatant in each well containing an annealed cDNA clone now contains amplified copies of that clone, and the library is normalized with respect to clone representation. The supernatants can be removed and PCR amplified again, while the wells retain unique single-stranded DNA that can be used as a template for future amplification reactions as desirable. This method allows the rapid isolation of clones from a normalized library and overcomes possible difficulties arising from the relative abundance of the desired clone(s), such as an odorant GPCR

### EXAMPLES

### Example 1: Identification, cloning, and characterization of the Anopheles gambiae odorant receptor responsible for detecting mating pheromone. Developing an Arometic compound capable of altering Anopheles behavior by mimicking the function of the naturally occurring pheromone.

This example relates to methods of cloning an odorant receptor gene and identifying ligands to the receptor it encodes. In this example, the odorant receptor that responds to mating pheromone is cloned, and ligands capable of binding it are identified. These ligands are capable of mimicking the naturally occurring *Anopheles* mating pheromone, and can be used to alter pest behavior by either prohibiting or interfering with members of one gender to find members of the other gender, or by eliminating or reducing the mating response. Thus, the present disclosure provides methods and compositions to achieve pest control *via* mating disruption:
Several thousand *Anopheles* mosquitoes are captured and separated into male and female genders (see **FIG. 7**). The antennae of each gender are dissected separately, and mRNA is isolated from each pool of antennae. The bodies of both gender are retained and mRNA is also extracted from them. cDNA is transcribed from each mRNA sample, to create three pools of cDNA (♂antennae cDNA, ♀ antennae cDNA and body cDNA). One portion of each cDNA pool is reserved to make labeled hybridization probes later, while the rest is used to construct cDNA libraries in a suitable vector; examples include but are not limited to bacterial plasmids or λ phage. This process yields three cDNA libraries; one library contains clones representing the transcripts from male antennae (♂antennae library #1), another library contains clones from mRNA representing the transcripts from female antennae (♀antennae library #1), and a third library contains clones representing the mRNA transcripts from the mosquito bodies (body library #1).

The libraries with clones from the antennae of both genders (♂antennae library #1 and ♀antennae library #1) are arrayed on nitrocellulose filters and screened with a labelled probe made from cDNA expressed in the bodies. Positive clones are optionally discarded in order to examine those clones specific to the antennae, since the odorant receptor responsible for identifying pheromones will tend to be expressed selectively in antennal tissues rather than elsewhere in the body. This produces ♂antennae library #2 and ♀ antennae library #2. The ♂antennae library #2 is screened with a probe derived from male antennal cDNA and negative clones are eliminated or reduced because they lack an insert (this produces ♂antennae library #3). The ♂antennae library #3 is then screened with a probe derived from female antennal cDNA and negative clones are selected, since they tend to represent transcripts expressed specifically in male antennae and not female antennae or bodies (yielding ♂antennae library #4). Clones from ♂antennae library #4 are sequenced and the data analyzed for the presence of regions encoding transmembrane repeats, a characteristic of odorant receptors.

The ♀antennae library #2 is screened with a probe derived from female antennal cDNA and negative clones are eliminated or reduced because they lack an insert (this produces ♀antennae library #3). The ♀antennae library #3 is then screened with a probe derived from male antennal cDNA and negative clones are selected or enriched, since they represent transcripts expressed specifically in female antennae and not male antennae or bodies (yielding ♀antennae library #4). Clones from ♀antennae library #4 are sequenced and the data analyzed for the presence of regions encoding transmembrane repeats, a characteristic of odorant receptors.

The present disclosure provides means for functional analysis of a receptor and for identifying potential ligands once an odorant receptor specific, for example, to the male antennae has been isolated. For example, the odorant receptor gene along with a reporter gene construct is transformed into a *Drosophila* or mammalian cell line to allow assaying the reporter's response to potential ligands *in vitro.* Combinatorial chemical libraries, either commercially available or constructed in a proprietary manner, are then screened to identify compounds capable of binding the expressed receptor. These lead compounds are evaluated in behavioral assays using *Drosophila* transformed with the odorant receptor gene; in these assays, fly behavior is observed and evaluated for a response to the presence of the lead compound. In parallel, the antennae of *Drosophila* transformed with the *Anopheles* odorant receptor gene are dissected and their response to the lead compound is evaluated using electroantennograms. Note that *Drosophila* mutants lacking endogenous odorant receptor gene expression are readily available and can be utilized in these experiments to minimize any potential cross-talk between the endogenous and transformed odorant receptor pathways. However, this may not be desirable since the lead compound is unlikely to stimulate behavioral responses in *Drosophila,* a species with a vastly different mating behavior, feeding behavior, and life cycle than *Anopheles.*

These procedures can yield a compound capable of altering the mating behavior of adult male *Anopheles* mosquitoes. This compound can have a number of applications, including use as an agent in pest management programs implementing mating disruption. Furthermore, unlike the native *Anopheles* pheromone, such a compound may have a relatively simple chemical composition that lends itself to manipulation in order to achieve desirable physical properties. That is, the compound's solubility, viscosity, pH, and other properties can be modified to make it more suitable for deployment.

### Example 2: Using protein-protein interactions in Phretcheck, an in vitro assay system, to rapidly identify the odorant receptor of the Glassy-winged Sharpshooter, Homalodisca coagulata.

The present disclosure provides a method to identify compounds capable of binding the odorant receptor of any species by taking advantage of the known protein-protein interaction between GPCRs and Gα protein. Gα proteins are conserved across species, making the isolation of the gene encoding the *Homalodisca* Gα protein feasible. For example, degenerate PCR primers based on consensus Gα gene sequences can be used with *Homalodisca* cDNA as template to generate a *Homalodisca-derived* Gα-specific cDNA probe, then screening the *Homalodisca* cDNA library with that probe to isolate cDNAs that encode the Gα. A reporter gene cascade comprising the Gα protein coupled to a FRET reporter mechanism (utilizing Green Fluorescent Protein, for example) is transformed into a mammalian or insect cell line. The reporter gene - Gα protein construct will be initially localized in the cytoplasm of transformed cells, but will migrate to the plasma membrane if it can interact with the *Homalodisca* odorant receptor. This migration is the basis of the Phretcheck assay described in this example.

A cDNA library from *Homalodisca* antennal tissues is constructed using a transformation vector. The cell line incorporating the Gα protein fusion construct is transformed with the cDNA library and the resulting double-transformant cells, already preloaded with DiI (a hydrophobic fluorescent dye specifically incorporated into cell membranes), are assayed for FRET under fluorescent microscopy or high-throughput cell sorting. FRET indicates the Gα protein fusion construct has migrated to the plasma membrane and is interacting with a potential GPCR. Cells exhibiting FRET are isolated and the transformation constructs sequenced to determine the identity of the potential GPCR.

### Example 3: Determining whether an orchard is infected with the Codling moth, Cydia pomonella Linnaeus using the Bioscensor device.

The present disclosure provides methods to isolate the odorant receptor responsible for pheromone detection from any species. The present disclosure also provides a method to incorporate the technology developed herein into a Bioscensor device capable of detecting the presence of pest species or other species of interest in the field. In this example, the Bioscensor device will detect the presence of the orchard pest, *Cydia pomonella Linnaeus, in situ -* that is, in an orchard being monitored for infestation.

In this example, the GPCR responsible for pheromone detection in *Cydia* is transformed into *Drosophila,* mammalian, yeast, or other eukaryotic cells incorporating a reporter gene cascade coupled to an ion channel. These cells are then embedded into silica gel capable of transmitting detectable current across two electrodes. The device is used in the field and indicates whether the vineyard is infected with *Cydia* by detecting the presence of that species' pheromone. If the pheromone is present in the atmosphere, the GPCRs on the plasma membranes of the cells embedded in the silica gel bind to it, initiating the signaling cascade that results in ion flux across the plasma membrane. This ion flux causes a change in electrical current or potential that can be measured.

Alternatively, an enzymatic reaction resulting in a color change on a strip can be used instead of ion flux for detecting pheromones. In this method, the GPCR is coupled to an enzymatic reporter cascade which, when evoked, releases a colored product (for example, β-galactosidase). The appearance of this product on the device indicates the presence of pheromone.

### Example 4: Determining the amount of artificially applied Codlemone in order to control Cydia pomonella Linnaeus populations.

The present disclosure provides methods to quantify the amount of artificially applied pheromone in the field. This is particularly useful when attempting to control insect pest populations using mating disruption based on pheromone application. After an initial pheromone application, the present disclosure can indicate when another application is desirable. In this example, the present disclosure is used to monitor the application of codling moth codlemone.

The GPCR responsible for codlemone detection in the codling moth is transformed into *Drosophila,* mammalian, yeast, or other eukaryotic cells incorporating a reporter gene cascade coupled to an ion channel. These cells are then embedded into silica gel capable of transmitting detectable current across two electrodes. If codling moth codlemone is present in the atmosphere, the GPCRs on the plasma membranes of the cells embedded in the silica gel bind to it, initiating the signaling cascade that results in ion flux across the plasma membrane. This ion flux causes a change in electrical current or potential that can be measured. In this manner, the present disclosure can be used to indicate whether further codlemone application is desirable.

Alternatively, an enzymatic reaction resulting in a color change on a strip can be used instead of ion flux for detecting codlemone. In this method, the GPCR is coupled to an enzymatic reporter cascade which, when evoked, releases a colored product (for example, β-galactosidase). The appearance of this product on the device indicates the presence of codlemone.

### Example 5: Developing a repellent for Anopheles gambiae mosquitoes.

The mosquito *Anopheles gambiae* is particularly threatening to humans since it carries a microbe responsible for malaria. None of the currently available mosquito repellents are effective against all *Anopheles* species, for reasons not sufficiently understood. the present disclosure provides a method to develop an effective repellent against *Anopheles gambiae* and against any insect with a known GPCR

Blood sucking insects like *Anopheles* use GPCRs to identify potential hosts. These GPCRs are expressed in the antennae, and female mosquitoes feed on blood. Thus, cDNA libraries from male antennae, female antennae, and bodies can be used according to the methods of the present disclosure to isolate and characterize the GPCR (referred to in this example as GPCR1) used by *Anopheles* to identify human hosts.

Antagonists to GPCR1 will block its ability to function, prohibiting the mosquitoes from identifying potential hosts and feeding. The present disclosure provides a method to identify antagonists once GPCR1 is isolated, by screening combinatorial chemical libraries. Identification of antagonists is accomplished using high-throughput assays of large combinatorial chemistry libraries to identify GPCR1 ligands. The GPCR1 ligands are then sorted into agonists and antagonists, depending on their effect on the receptor, which can be assayed by techniques common in the art. Effective antagonists can then be incorporated into a water or oil based repellent lotion, or devices that can be worn on animals or humans. An example of the latter device is a mosquito repellent wristband or necklace for humans or animals. Nets, fabrics, and powders can also incorporate the novel repellent.

### Example 6: Identification, cloning, and characterization of Anopheles gambiae odorant-binding proteins. Developing an OBP-binding compound capable of altering Anopheles behavior by inhibiting OBP-binding protein function. Developing a novel form of insect repellent.

*Anopheles gambiae* mosquitoes are relatively widespread and are known to harbor the infectious agent responsible for malaria in humans. In general, previous efforts to control *Anopheles* populations have been based on toxic pesticides. The present disclosure provides the compositions and methods desirable to clone and characterize odorant-binding proteins responsible for efficient olfaction in *Anopheles.* Since this species relies on olfactory information to find mates and humans on which to feed, the present disclosure provides compositions and methods to develop non-toxic pest control products. For example, characterizing *Anopheles* OBPs could provide the information desirable to develop a pest control approach based on mating disruption or another form of behavior alteration, since successful mating depends on the male *Anopheles* locating a female ready to mate. Another feasible approach using the compositions and methods provided by the present disclosure is to develop a pest control approach based on rendering *Anopheles* incapable of detecting the scent of humans. In this manner, products developed based on the compositions and methods provided by the present disclosure will replace current insect repellents.

The present disclosure provides methods of cloning genes encoding odorant-binding proteins and subsequently identifying compounds or chemicals capable of binding the OBPs in order to block their normal function. By inducing anosmia, these compounds can be used to alter pest behavior by prohibiting members of one gender to find members of the other gender, eliminating the mating response entirely, or prohibiting pests from locating humans on which to feed.

Several thousand *Anopheles* mosquitoes are separated into male and female genders. The antennae of each gender are dissected separately, and mRNA is isolated from each pool of antennae. mRNA is also extracted from the bodies of both genders. cDNA is transcribed from each mRNA sample. One portion of the extracted cDNA is reserved in order to make labeled hybridization probes later, while the rest is used to construct cDNA libraries in a suitable vector; examples include but are not limited to bacterial plasmids or λ phage. This process yields three cDNA libraries; one library contains clones representing the transcripts from male antennae, another library contains clones from mRNA representing the transcripts from female antennae, and a third library contains clones representing the mRNA transcripts from the mosquito bodies.

The libraries with clones from the antennae of both genders are arrayed on nitrocellulose filters and screened with a labeled probe made from cDNA expressed in the bodies. Positive clones are discarded in order to examine only those clones specific to the antennae, since the genes encoding OBPs will be expressed selectively in antennal tissues rather than elsewhere in the body. The female antennal library is screened with a probe derived from female antennal cDNA and negative clones are eliminated or reduced because they lack an insert. Highly expressed clones are sequenced and the data analyzed for the presence of cDNAs encoding OBPs. Approximately 15% of all antennal cDNAs were found to encode OBPs.

The present disclosure provides compositions and methods for functional analysis of an OBP and for identifying compounds capable of binding an OBP optionally with high affinity. Combinatorial chemical libraries, either commercially available or constructed in a proprietary manner, are screened to identify compounds capable of binding OBPs expressed *in vitro,* and these lead compounds are evaluated in behavioral assays to determine whether they are capable of blocking normal responses to odors and pheromones in *Anopheles* adults.

These procedures will yield a compound capable of altering the mating and feeding behaviors of adult *Anopheles* mosquitoes. Such a compound can have a number of applications, including use as an agent in pest management programs implementing mating disruption or in repellent products designed to make humans undetectable or reduce detectability by mosquitoes searching for a blood meal. Furthermore, such a compound can have a relatively simple chemical composition that lends itself to manipulation in order to attain desirable physical properties. For example, the compound's viscosity, pH, solubility, and other properties can be modified to make it more suitable for deployment in a range of environments, or as an agent in a variety of products. The compound may thus be used in bracelets or necklaces, or in bed netting, fabrics, powders, gels, liquids, or emulsions.

### Example 7: Developing a mosquito attractant to trap and kill female Culex mosquitoes.

Insect traps have traditionally relied on a compound attractive to a target species or range of species that lures the insects to the trap, combined with a toxin or physical barrier (such as an adhesive) that results in death for trapped insects. The present disclosure provides compositions and methods to develop species-specific mosquito attractant compounds for use in traps. In this example, female *Culex* mosquitoes are selectively lured using a compound developed by the present disclosure.

Combinatorial chemical libraries are used as shown in **FIG. 7** to identify and isolate compounds capable of interacting with olfactory molecules (for example, OBPs or GPCRs) specifically expressed or expressed in female *Culex.* A variety of techniques discussed in this patent application, including electroantennograms, can be used to identify those compounds that can effectively mimic the scent of blood meals. These compounds can then be incorporated into traps that specifically attract female *Culex* mosquitoes and kill them by using a toxin, immobilizing them, or electrocuting them.

### Example 8: Developing an odor control product for use in public lavatories.

Public lavatories in locations such as train stations, large cinema complexes, docks or ports, and airports are subject to frequent use by a large number of people in transit, and can often develop unpleasant odors. Extensive research has been devoted to developing products to mask such odors; current commercially available products typically attempt to mask the offending odors by emanating an even stronger scent of their own. Regrettably, this chemical scent is often also unpleasant and many patrons find the combined odors of the lavatories, cleansers, refuse, and odor control products rather overbearing. The present disclosure provides methodology and compositions to develop effective odor-masking products that operate by reducing patrons' olfactory sensitivity rather than attempting to overpower an unpleasant odor by emanating an even stronger odor of their own. These odor-masking products can be incorporated into cleansers in an aqueous solution or suspension, or in solid, crystal or granular form.

For example, human OBPs are isolated using methods similar to the ones shown in **FIG. 7**. DNA sequences encoding these human OBPs are expressed *in vitro* using tools common in the art, and their protein products are used to screen combinatorial chemical libraries. The goal of these screens is to isolate compounds capable of interacting with the OBPs that also exhibit desirable chemical, biological, and physical characteristics. These non-toxic, relatively simple compounds are capable of strongly binding human OBPs and can thus be used to block OBP function. The compounds are incorporated into the cleansers used to clean the lavatories, the air fresheners used to reduce odors in the lavatories, or in the building materials (tiles, dry wall, etc.) used to construct the lavatories. In this manner, the present disclosure provides for compounds that manipulate the sensitivity of human olfaction and can be used to control unpleasant odors in any sort of environment where such a need arises routinely.

### Example 9: Using OBPs or their peptide derivatives to develop gels that can selectively remove odors from the environment.

The present disclosure provides the compositions and methods desirable to develop highly effective products to inhibit odors. These products can be used in any environment where unpleasant odors need to be contained. To develop these products, odorant-binding proteins can be purified, enriched or identified using the methods provided by the present disclosure.

For example, DNA sequences encoding OBPs or peptide derivatives are expressed *in vitro* using tools common in the art. The OBPs or peptide derivatives are then incorporated into aqueous gels that can be deployed in environments where odor control is desirable. Odors, scents, or semiochemicals emanating from those environments will be bound by the OBP, or peptide derivatives of the OBP, that essentially act as odor traps. Detectable odors will thus be greatly reduced. These odor control gels incorporating OBPs or OBP peptide derivatives are useful in locations including but not limited to private or public lavatories, garages, kitchens, storage areas, or refuse containers.

### Example 10: Rapidly isolating genes involved in Aedes aegypti olfaction, using a normalized cDNA library generated from antennal tissue.

The mosquito *Aedes aegypti* harbors the infectious agent responsible for Yellow Fever in humans, and poses a serious public health threat in many regions of the world. Until now, attempts to control *Aedes* populations utilized highly toxic pesticides, usually with mixed results. Thus, several laboratories are studying *Aedes* biology in an effort to develop tools that control the mosquito more effectively.

The present disclosure recognizes that characterizing the molecules involved in *Aedes* olfaction could provide the information desirable to develop a pest control approach based on behavior alteration or to design an effective repellent. Traditional approaches to screening cDNA libraries for novel gene transcripts have been hindered by the complexity of the libraries and the relative abundance of the transcripts. In this example, the present disclosure provides the means and constructs for cloning the genes encoding rare olfactory proteins rapidly and efficiently by using an arrayed, normalized cDNA library. The example utilizes a library containing clones representing transcripts expressed in the antennae of male *Aedes* mosquitoes, and shows how rare transcripts are isolated from the complex cDNA library.

During library construction, a 5' polylinker region useful as a site for priming PCR reactions is introduced into every clone. This region may contain a host of restriction endonuclease recognition sites as desired. The completed library is arrayed into multi-well plates, each well containing a collection of 16 oligonucleotides anchored via their 5' end to a glass bead. The anchored oligonucleotides are present in nanomolar concentrations allowing their use as primers in subsequent PCR reactions. **FIG. 5** depicts the experimental design and the sequence variations introduced in each oligonucleotide set. The diversity of the anchored oligonucleotides in the array as a whole is sufficient to ensure that every possible cDNA sequence can be bound by an individual, specific oligonucleotide in a single well.

Clones from the cDNA library to be screened are heated to denature them into single-stranded DNA and annealed to the anchored primers in the wells of the array plates. Several washes remove unbound cDNAs from each well, leaving only the newly-annealed DNA duplexes consisting of the anchored oligonucleotides and any specifically bound cDNA clone behind in each well. PCR reactions using the anchored oligonucleotides as a 3' primer and a 5' primer derived from the 5' sequence shared by every cDNA clone in the library can be performed directly in the wells of the array plates. These PCR reactions only amplify those clones that are bound by an anchored oligonucleotide in each well. These methods and constructs result in a library that is normalized with respect to clone representation. The supernatants can be removed and PCR amplified again, while the wells retain unique single-stranded DNA that can be used as a template for future amplification reactions as desirable. In this manner, relatively rare transcripts can easily be isolated from any cDNA library regardless of the library's initial complexity. Sequence data analysis using bioinformatics tools common in the art can now be used to identify homologs of known genes and genes encoding novel products alike.

### Example 11: Protecting a vineyard from infection by the Glassy-winged sharpshooter, Homalodisca coagulata, using novel repellents or attractants.

The Glassy-winged sharpshooter, *Homalodisca coagulata,* poses a serious threat to citrus and vineyards in California and elsewhere. The present disclosure recognizes the need to develop novel, highly effective products to control this insect pest, and provides methods and compositions to do so. These products can generally be classified as either repellents or attractants; both classes of product are based on compounds isolated from combinatorial chemical libraries based on their ability to interact with insect olfactory proteins, including OBPs and GPCRs. Thus, the initial steps involving screening combinatorial chemical libraries for compounds capable of interacting with olfactory proteins from *Homalodisca* are common to the development of either a repellent or an attractant. A brief example of each implementation follows.

Repellents: Compounds capable of activating olfactory pathways controlling aversive reactions or compounds capable of inducing anosmia can be incorporated into repellents. These repellents can be deployed as aerosols, gels, or sprays to coat vines or plants, or as powders or solids. These repellents will prevent the insects from locating food or mates, or generate a distasteful odor to drive the insects away from the fields being protected.

Attractants: Compounds capable of attracting either or both sexes of *Homalodisca* can be incorporated into attractants. These attractants can be used to construct traps or lures in a bait-and-kill pest control scheme, where the insects are attracted to a toxin, a source of electricity, or a source of heat that subsequently kills them, or a trap that immobilizes them.

### Example 12: Developing silverfish and firebrat repellents that can be incorporated into solids in order to control pests such as Lepisma saccharina and Thermobia domestica.

The common silverfish, *Lepisma saccharina,* and the firebrat, *Thermobia domestica,* are similarly shaped, relatively primitive insect pests of the order *Thysanura* that consume and/or masticate material high in protein, sugar, or starch. Their target foods can include cereals, flour, books, paper, glue, wallpaper, cotton, linen, silk, rayon, and paste, making them a significant domestic, agricultural, and commercial nuisance.

The present disclosure recognizes the need to develop effective repellents against these pests, and provides the means and compositions desirable to isolate and identify compounds with desirable effects and chemical or physical properties to be incorporated into these repellents. Desirable properties include but are not limited to the ability to be integrated in packing materials (paper, cardboard, plastics, or fabrics) and building materials.

Repellents are composed of molecules or compounds isolated from combinatorial chemical libraries based on their ability to interact with odorant proteins (for example, OBPs and GPCRs) from *Lepisma* or *Thermobia* using the means and methods of the present disclosure. Briefly, sensory tissue (antennae) are dissected and tissue-specific cDNA libraries of clones representing the genes expressed in antennal tissue are constructed using the techniques described herein. These libraries are screened for clones encoding OBPs or GPCRs, using methods described herein; the genes identified are expressed *in vitro* and combinatorial chemical libraries are screened for compounds that interact with the OBP or GPCR in question. Compounds capable of activating olfactory pathways controlling aversive reactions or compounds capable of inducing anosmia can be incorporated into repellents; the effect of such compounds can be verified by conducting electrophysiological experiments, including electroantennograms, or behavioral assays using either *Lepisma* and *Thermobia* specimens , using methods described herein. Thus, the present disclosure provides the means and compositions to develop a pest repellent incorporated into cardboard, fabric, or paper to protect stored foods, or a repellent to protect paper products, building materials, structural materials, fabrics, and storage materials.

### Example 13: Developing and deploying effective termite control products for domestic and commercial use.

Termites (for example, *Reliculotermes spp*.) are isopteran insects that cause extensive damage to domestic and commercial structures by attacking wood. Most previous attempts to control termites relied on toxins, including arsenic compounds, either applied after construction or incorporated into the wood itself via pressure treatment. Environmental and public health concerns have limited the toxins available to exterminators, while existing homes and commercial structures are aging and new homes and structures are being built with lumber from younger and younger trees that is more susceptible to termite infestation.

The present disclosure provides the methods and compositions desirable to develop novel, nontoxic termite control products. These products can be repellents or attractants; both classes of product are based on compounds or molecules isolated from combinatorial chemical libraries based on their ability to interact with termite olfactory proteins, including OBPs and GPCRs, as described herein. Therefore, the initial steps - screening combinatorial chemical libraries for compounds capable of interacting with olfactory proteins from *Reticulotermes -* are common to the development of either a repellent or an attractant. A brief example of each implementation follows.

Repellents: Compounds capable of activating olfactory pathways controlling aversive reactions or compounds capable of inducing anosmia can be incorporated into repellents. These repellents can be incorporated into lumber using techniques similar to those employed currently to incorporate arsenic compounds. These repellents will prevent termites from locating treated wood, or generate a distasteful odor to drive the insects away from the buildings, homes, or structures being protected.

Attractants: Compounds capable of attracting *Reticulotermes* can be incorporated into attractants. These attractants can be used to construct traps or lures in a bait-and-kill pest control scheme, where the termites are attracted to a toxin (for example, an arsenic compound), a source of electricity, or a source of heat that subsequently kills them, or a trap that immobilizes them.

### Example 14: Developing products to control the common rabbit, Oryctolagus cuniculus.

Mammals are an often overlooked but nevertheless significant pest category. For example, lagomorphs, like the common rabbit *Oryctolagus cuniculus,* cause considerable damage to fruit trees and crops ranging from grains and vines to vegetables. Traditional rabbit control methods include electric fences, poisons, and shooting. However, rabbits proliferate prodigiously and are thus extremely difficult to eradicate. The present disclosure recognizes the need to develop effective, efficient, and humane mammalian pest control products, and provides the methods and compositions necessary to do so. These products can be repellents or attractants; both classes of product are based on compounds or molecules isolated from combinatorial chemical libraries based on their ability to interact with pest olfactory proteins, including OBPs and GPCRs, as described herein. The initial development steps - screening combinatorial chemical libraries for compounds capable of interacting with olfactory proteins from the pest species - are common to the development of either a repellent or an attractant. Each implementation is developed as follows:
Repellents: Compounds capable of activating olfactory pathways controlling aversive reactions or compounds capable of inducing anosmia can be incorporated into repellents. These repellents can be incorporated into aerosol sprays, liquid solutions, or solids and deployed in fields or anywhere else rabbit control is desired by spraying, scattering, or incorporation into repellent stations. These repellents will prevent rabbits from locating treated areas, or generate a distasteful odor to drive the pests away from the fields, farms, homes, or areas being protected.
Attractants: Compounds capable of attracting *Oryctolagus* can be incorporated into attractants. These attractants can be used to construct traps or lures useful to farmers and hunters alike.

### Example 15: Isolating and characterizing an OBP, and using a surface plasmon resonance detector to identify potential Arometics for use in controlling the kissing bug, Triatoma.

The present disclosure recognizes the need to rapidly develop pest control products. The present disclosure thus provides methods and compositions to do so, and further recognizes that an automated process to identify Arometics can greatly accelerate product development. Toward this end, the present disclosure incorporates use of a commercially available, robotic surface plasmon resonance detector (for example, the apparatus constructed by Biacore AG, Sweden) in screening combinatorial libraries for Arometics lead molecules. In this example, the present disclosure provides the methods and compositions desirable to isolate OBPs from the kissing bug, *Triatoma,* a harmful species responsible for widespread illness and death in central and south America:
Animals are collected and antennae are dissected and frozen on dry ice; the bodies of each gender are also frozen. The tissue samples are then separately used to isolates mRNA, which is transcribed *in vitro* and used to construct cDNA libraries in a suitable vector. These cDNA libraries represent the genes expressed in the sensory organs and in the bodies. The libraries can be arrayed and screened for transcripts that are specifically expressed in the antennae. This is accomplished as follows:
   Arrayed clones are plated on nitrocellulose or nylon filters suitable for hybridization and screened with probes made from mRNA that is isolated from bodies lacking antennae. Positive clones are discarded in order to select for clones representing RNA expressed specifically in the antennae. The pooled antennal cDNA is used to make 5probes representing all RNA expressed in antennae. The library is screened with the probe to eliminate vectors lacking an insert.

This screening process results in the isolation of tissue-specific clones; all clones isolated are present only in one tissue type, namely, the antennal tissues where OBPs are normally expressed. All of the isolated clones are sequenced. Clones encoding potential OBPs can be identified utilizing on bioinformatics analysis common in the art.

OBPs are then expressed *in vitro,* purified, and individually bound to a Biacore chip as ligands. Combinatorial chemical libraries are screened for compounds capable of interacting with the OBPs by testing the chemical libraries as analytes over the chip-bound OBP using a Biacore apparatus.

### REFERENCES

Ashburner, M., Drosophila: A Laboratory Manual (1989) Cold Spring Harbor Laboratory Press, Cold Spring Harbor, N.Y., USA.
Sambrook et al., Molecular Cloning: A Laboratory Manual, 2nd Ed. (1989) Cold Spring Harbor Laboratory Press, Cold Spring Harbor, N.Y, USA.
Adams, M., Celniker, S., Holt, R. A., Evans, C. A., Gocayne, J. D., Amanatides, P. G., Scherer, S. E., Li, P. W., Hoskins, R. A., Galle, R. F.; et al. (2000) The genome sequence of Drosophila melanogaster. Science 287: 2185-2195.
Altschul, S. F., Madden, T. L., Alejandro A. Schäffer, A. A., Zhang, J., Zhang, Z., Miller, W. and Lipman, D. J. (1997) Gapped BLAST and PSI-BLAST: a new generation of protein database search programs. Nucleic Acids Res. 25: 3389-3402.
Anholt, R. R., Lyman, R. F. and Mackay, T. F. C. (1996) Effects of single P-element insertions on olfactory behavior in Drosophila melanogaster. Genetics 143: 293-301.
Arca, B., Lombardo, F., de Lara Capurro, M., Della Torre, A., Dimopoulos, G., James, A. A. and Coluzzi, M. (1999) Trapping cDNAs encoding secreted proteins from the salivary glands of the malaria vector Anopheles gambiae. Proc. Natl. Acad. Sci. USA 96: 1516-1521.
Bhalla, S. C. (1968) White eye, a new sex-linked mutant of Aedes aegypti. Mosquito News 28: 380-385.
Biessmann, H., Kobeski, F., Walter, M. F., Kasravi, A. and Roth, C. W. (1998) DNA organization and length polymorphism at the 2L telomere of Anopheles gambiae. Insect Mol. Biol. 7: 83-93.
Breer, H., Krieger, J. and Raming, K. (1990) A novel class of binding proteins in the antennae of the silk moth Antheraea perneyi. Ins. Biochem. 20: 735-740.
Carlson, J. R. (1996) Olfaction in Drosophila: from odor to behavior. Trends Genet. 12: 175-180.
Christophides, C. K., Mintzas, A. C. and Komitopoulou, K. (2000) Organization, evolution and expression of a multigene family encoding putative members of the odourant binding protein family in the medfly Ceratitis capitata. Ins. Mol. Biol. 9: 185-195.
Clyne, P. J., Warr, C. G., Freeman, M. R, Lessing, D., Kim, J. and Carlson, J. R. (1999) A novel family of divergent seven-transmembrane proteins: candidate odorant receptors in Drosophila. Neuron 22: 327-338.
Cork, A. and Park, K. C. (1996) Identification of electrophysilogically-active compounds for the malaria mosquito, Anopheles gambiae, in human sweat extracts. Med. Vet. Entomol. 10: 269-276.
de Jong, R. and Knols, B. G. J. (1996). Selection of biting sites by mosquitoes. In "Olfaction in mosquito-host interactions. Ciba Foundation Symposium 200" (J. G. Hildebrand, Ed.), pp. 89-99. John Wiley &Sons, Chichester.
Du, G., Ng, C.-S. and Prestwich, G. D. (1994) Odorant binding by a pheromone binding protein: active site mapped by photoaffinity labeling. Biochem. 33: 4812-4819.
Du, G. and Prestwich, G. D. (1995) Protein structure encodes the ligand binding specificity in pheromone binding proteins. Biochem. 34: 8726-8732.
Feng, L. and Prestwich, G. D. (1997) Expression and characterization of Lepidopteran general odorant binding protein. Ins. Biochem. Mol. Biol. 27: 405-412.
Field, L. M., Pickett, J. A. and Wadhams, L. J. (2000) Molecular studies in insect olfaction. Ins. Mol. Biol. 9: 545-551.
Foster, W. A. (1995) Mosquito suger feeding and reproductive energetics. Ann. Rev. Entomol. 40: 443-474.
Gao, Q. and Chess, A. (1999) Identification of candidate Drosophila olfactory receptors from genomic DNA sequence. Genomics 60: 31-39.
Georghiou, G. P. and Wirth, M. C. (1997) Influence of exposure to single versus multiple toxins of Bacillus thuringiensis subsp. israelensis on development of resistance in the mosquito Culex quinquefasciatus (Diptera: Culicidae). Appl. Environ, Microbiol. 63: 1095-1101.
Githeko, A. K., Brandling-Bennett, A. D., Beier, M., Atieli, F., Owaga, M. and Collins, F. H. (1992) The reservoir of Plasmodium falciparum malaria in a holoendemic area of western Kenia. Trans. R. Soc. Med. Hyg. 86: 355-358.
Graham, L. A., Tang, W., Baust, J. G., Liou, Y.-C., Reid, S. and Davies, P. L. (2001) Characterization and cloning of a Tenebrio molitor hemolymph protein with sequence similarity to insect odorant-binding proteins. Ins. Biochem. Mol. Biol. 31: 691-702.
György, T. K., Roby-Shemkowitz, A. J. and Lerner, M. R. (1988) Characterization and cDNA cloning of the pheromone binding protein from the tobacco hornworm, Manduca sexta: a tissue-specific developmentally regulated protein. Proc. Natl. Acad. Sci. USA 85: 9851-9855.
Hekmat-Scafe, D. S., Dorit, R. L. and Carlson, J. R. (2000) Molecular evolution of odorant-binding protein genes OS-E and OS-F in Drosophila. Genetics 155: 117-137.
Hildebrand, J. G. (1996). Olfaction in mosquito-host interactions. Ciba Foundation Symposium 200. John Wiley & Sons, Chichester.
James, A. A., Blackmer, K., Marinotti, O., Ghosn, C. R. and Racioppi, J. V. (1991) Isolation and characterization of the gene expressing the major salivary gland protein of the female mosquito, Aedes aegypti. Mol. Biochem. Parasitol. 44: 245-253.
Kim, M.-S., Repp, A. and Smith, D. P. (1998) LUSH odorant-binding protein mediates chemosensory responses to alcohols in Drosophila melanogaster. Genetics 150: 711-721.
Kline, D. L. (1999) Olfactory responses and field attraction of mosquitoes to volatiles from limburger cheese and human foot odor. J. Vector Ecol. 23: 186-194.
Kline, D. L., Takken, W. and Carlson, D. A. (1990) Field studies on the potential of butanone, carbon dioxide, honey extract, 1-octen-3-ol, L-lactic acid and phenols as attractants for mosquitoes. Med. Vet. Entomol. 4: 383-391.
Knols, B. G. J., van Loon, J. J. A., Cork, A., Robinson, R. D., Adam, W., Meijerink, J., DeJong, R. and Takken, W. (1997) Behavioural and electrophysiological responses of the female malaria mosquito Anopheles gambiae (Diptera: Culicidae) to Limburger cheese volatiles. Bull. Entomol. Res. 87: 151-159.
Kodrik, D., Filippov, V. A., Filippova, M. A. and Sehnal, F. (1995) Sericotropin: an insect neurohormonal factor affecting RNA transcription. Nether. J. Zool. 45: 68-70.
Krieger, J. and Breer, H. (1999) Olfactory reception in invertebrates. Science 286: 720-723.
Krieger, J., Gänssle, H., Raming, K. and Breer, H. (1993) Odorant binding proteins of Heliothis virescens. Ins. Biochem. Mol. Biol. 23: 449-456.
Krieger, J., Raming, K., Prestwich, G. D., Frith, D., Stabel, S. and Breer, H. (1992) Expression of a pheromone-binding protein in insect cells using a baculovirus vector. Eur. J. Biochem. 203: 161-166.
Krieger, J., von Nickisch-Rosenegk, E., Mamali, M., Pelosi, P. and Breer, H. (1996) Binding proteins from the antennae of Bombyx mori. Ins. Biochem. Mol. Biol. 26: 297-307.
Maibeche-Coisné, M., Longhi, S., Jacquin-Joly, E., Brunel, C., Egloff, M.-P., Gastinel, L., Cambiilau, C., Tegoni, M. and Nagan-Le Maillour, P. (1998) Molecular cloning and bacterial expression of a general odorant-binding protein from the cabbage armyworm Mamstra brassicae. Eur. J. Biochem. 258: 768-774.
Maleszka, R. and Stange, G. (1997) Molecular cloning, by a novel approach, of a cDNA encoding a putative olfactory protein in the labial palps of the moth Cactoblastis cactorum. Gene 202: 39-43.
Mboera, L. E. G. and Takken, W. (1997) Carbon dioxide chemotropism in mosquitoes (Diptera: Culicidae) and its potential in vector surveillance and management. Rev. Med. Vet. Entomol 85: 355-368.
5McIver, S. B. (1982) Sensilla of mosquitoes (Diptera: Culicidae). J. Med. Entomol. 19: 489-535.
McKenna, M. P., Hekmat-Scafe, D. S., Gaines, P. and Carlson, J. R. (1994) Putative Drosophila pheromone-binding proteins expressed in a subregion of the olfactory system. J. Biol. Chem. 269: 16340-16347.
Mombaerts, P. (1999) Seven-transmembrane proteins as odorant and chemosensory receptors. Science 286: 707-711.
Oduol, F., Xu, J., Niaré, O., Natarajan, R. and Vernick, K. D. (2000) Genes identified by an expression screen of the vector mosquito Anopheles gambiae display differential molecuar immune response to malaria parasites and bacteria. Proc. 5 Natl. Acad. Sci. USA 97: 11397-11402.
Paesen, G. C. and Happ, G. M. (1995) The B proteins secreted by the tubular accessory sex glands of the male mealworm, tenebrio molitor, have sequence similarity to moth pheromone-binding proteins. Ins. Biochem. Mol. Biol. 25: 401-408.
Pikielny, C. W., Hasan, G., Rouyer, F. and Rosbash, M. (1994) Members of a family of 5 Drosophila putative odorant-binding proteins are expressed in different subsets of olfactory hairs. Neuron 12: 35-49.
Prestwich, G. D., Du, G. and LaForest, S. (1995) How is pheromone specificity encoded in proteins? Chem. Senses 20: 461-469.
Rai, K. S. and Black IV, W. C. (1999) Mosquito genomes: structure, organization, and evolution. Adv. Genet. 41: 1-33.
Raming, K., Krieger, J. and Breer, H. (1989) Molecular cloning of an insect pheromone-binding protein. FEBS Let. 356: 215-218.
Robertson, H. M., Martos, R., Sears, C. R., Todres, E. Z., Walden, K. K. O. and Nardi, J. B. (1999) Diversity of odourant binding proteins revealed by an expressed 5 sequence tag project on male Manduca sexta moth antennae. Ins. Mol. Biol. 8: 501-518.
Rothemund, S., Liou, Y-C., Davies, P. L., Krause, E. and Sönnichsen, F. D. (1999) A new class of hexahelical insect proteins revealed as putative carriers of small hydrophobic ligands. Structure 7: 1325-1332.
Sandler, B. H., Nikonova, L., Leal, W. S. and Clardy, J. (2000) Sexual attraction in the silkworm moth: structure of the pheromone-binding protein-bombykol complex. Chem. Biol. 7: 143-151.
Smith, D. P. (1996) Olfactory mechanisms in Drosophila melanogaster. Curr. Op. Neurobiol. 6: 500-505..
Sutcliffe, J. F. (1994) Sensory bases of attractancy: morphology of mosquito olfactory sensilla-a review. J. Am. Mosq. Control Assn. 10: 309-315.
Takken, W. (1991) The role of olfaction in host-seeking of mosquitoes: a review Insect Sci. Applic. 12: 287-205.
Takken, W. and Knols, B. G. J. (1999) Odor-mediated behavior of afrotropical malaria mosquitoes. Annu. Rev Entomol. 44: 131-157.
Thymianou, J. D., Mavroidis, M., Kokolakis, G., Komitopoulou, K., Zacharopoulou, A. and Mintzas, A. C. (1998) Cloning and characterization of a cDNA encoding as male-specific serum protein of the Mediterranean fruit fly, Ceratitis capitata, with sequence similarity to odourant binding proteins. Ins. Mol. Biol. 7: 345-353.
Vogt, R. G., Prestwich, G. D. and Riddiford, L. M. (1988) Sex pheromone receptor proteins: visualization using a radiolabeled photoaffinity analog. J. Biol. Chem. 263: 3952-3959.
Vogt, R. G. and Riddiford, L. M. (1981) Pheromone binding and inactivation by moth 5 antennae. Nature 293: 161-163.
Vogt, R. G., Rybczynski, R. and Lerner, M. R. (1991) Molecular cloning of general odoramt-binding proteins GOBP1 and GOBP2 from the tobacco hawk moth Manduca sexta: comparisons with other insect OBPs and their signal peptides. J. Neurosci. 11: 2972-2984.
Vosshall, L. B., Amrein, H., Morozov, P.S., Rzhetsky, A. and Axel, R. (1999) A spatial map of olfactory receptor expression in the Drosophila antenna. Cell 96: 725-736.
Vosshall, L. B., Wong, A. M. and Axel, R. (2000) An olfactory sensory map in the fly brain. Cell 102: 147-159.
Watson, K. L., Konrad, K. D., Woods, D. F. and Bryant, P. J. (1992) The Drosophila homolog of the human S6 ribosomal protein is required for tumor suppression in the hematopoietic system. Proc. Natl. Acad. Sci. USA 89: 11302-11306.

## Claims

1. A method of reducing a target animal's sensitivity to odors, comprising :
a) providing a compound known to interact with OBPs of a target animal ;
b) incorporating said compound into products capable of altering said target animal behavior; and
c) exposing said target animal to the product containing said compound.

2. The method according to claim 1, wherein said odors are used to locate hosts.

3. The method according to claim 2, wherein said hosts are humans.

4. The method according to any one of claims 1 to 3, wherein said animal is an invertebrate.

5. The method according to claim 4, wherein said invertebrate is selected from the group consisting of dipterans, mosquitoes, gnats, flies, termites, lepidopterans, moths, butterflies, orthopterans, grasshoppers, locusts, sharpshooters, *Homalodisca spp*.,cockroaches, beetles, ants, fleas, silverfish, booklice, firebrants, hymenopterans, wasps, bees, hornets, kissing bugs, *Triatoma dimidiatamyria,* other insects, myriapods, millipedes and centipedes, mites, spiders, ticks, other arachnids, terrestrial isopods, pill bugs and sow bugs, other arthropods, annelids, nematodes, mollusks, snails and slugs.

6. The method according to claim 5, wherein said invertebrate is a mosquito.

7. The method according to any one of claims 1 to 3, wherein said animal is a vertebrate.

8. The method according to claim 7, wherein said vertebrate is selected from the group consisting of rodents, lagomorphs, insectivora, moles and shrews, chiroptera, carnivora, weasels, coyotes, bears, dogs and cats, artiodactyls, perissodactyls, primates, other mammals, reptiles, marine vertebrates including agnatha, chondrichthyes, sharks, osteichthyes, aves and pigeons.

## Patentansprüche

1. Verfahren zum Reduzieren der Geruchsempfindlichkeit eines Zieltiers, umfassend folgende Schritte:
a) Bereitstellen einer Zusammensetzung, von der bekannt ist, dass sie Wechselwirkungen mit den OBP (geruchsbindenden Eiweißen) eines Zieltiers hat;
b) Integrieren der Zusammensetzung in Produkte, die in der Lage sind, das Verhalten des Zieltiers zu verändern; und
c) Aussetzen des Zieltiers an das Produkt, das die Zusammensetzung umfasst.

2. Verfahren nach Anspruch 1, wobei die Gerüche verwendet werden, um Wirte ausfindig zu machen.

3. Verfahren nach Anspruch 2, wobei die Wirte Menschen sind.

4. Verfahren nach einem der Ansprüche 1 bis 3, wobei das Tier ein wirbelloses Tier ist.

5. Verfahren nach Anspruch 4, wobei das wirbellose Tier aus der Gruppe gewählt wird, die aus Zweiflüglern, Moskitos, Steckmücken, Fliegen, Termiten, Faltern, Motten, Schmetterlingen, Springschrecken, Grashüpfern, Heuschrecken, Zikaden, Homalodisca spp., Küchenschaben, Käfern, Ameisen, Silberfischen, Buchläusen, Ofenfischchen, Hautflüglern, Wespen, Bienen, Hornissen, Raubwanzen, Triatoma dimidiatamyria, anderen Insekten, Myriapoden, Doppelfüßern und Steinkriechern, Milben, Spinnen, Zecken und anderen Spinnentieren, terrestrischen Asseln, Kugelasseln und Kellerasseln, anderen Gliederfüßern, Ringelwürmern, Fadenwürmern, Weichtieren, Schnecken und Nacktschnecken besteht.

6. Verfahren nach Anspruch 5, wobei das wirbellose Tier ein Moskito ist.

7. Verfahren nach einem der Ansprüche 1 bis 3, wobei das Tier ein Wirbeltier ist.

8. Verfahren nach Anspruch 7, wobei das Wirbeltier gewählt wird aus der Gruppe, die aus Nagetieren, Hasentieren, Insektenfressern, Maulwürfen und Spitzmäusen, Fledermäusen, Fleischfressern, Wieseln, Kojoten, Bären, Hunden und Katzen, Paarhufern, Unpaarhufern, Primaten, anderen Säugetieren, Reptilien, Wasserwirbeltieren, zu denen Kieferlose, Knorpelfische, Haie, Knochenfische, Vögel und Tauben gehören, besteht.

## Revendications

1. Procédé de réduction de la sensibilité aux odeurs d'un animal cible, comprenant :
a) la fourniture d'un composé connu pour interagir avec les OBP d'un animal cible ;
b) l'incorporation dudit composé dans des produits pouvant altérer le comportement dudit animal cible ; et
c) l'exposition dudit animal cible au produit contenant ledit composé.

2. Procédé selon la revendication 1, dans lequel lesdites odeurs sont utilisées pour localiser des hôtes.

3. Procédé selon la revendication 2, dans lequel lesdits hôtes sont des humains.

4. Procédé selon l'une quelconque des revendications 1 à 3, dans lequel ledit animal est un invertébré.

5. Procédé selon la revendication 4, dans lequel ledit invertébré est sélectionné parmi le groupe consistant aux diptères, moustiques, moucherons, mouches, termites, lépidoptères, mites, papillons, orthoptères, sauterelles, criquets, cicadelles, Homalodisca spp., cafards, scarabées, fourmis, puces, poissons d'argent, procoptères, fourmis rouges (« firebrants »), hyménoptères, guêpes, abeilles, frelons, triatomas, Triatoma dimidiatamyria, autres insectes, mille-pattes, millipèdes et centipèdes, mites, araignées, tiques, autres arachnides, isopodes terrestres, cloportes et cloportes communs, autres arthropodes, annélides, nématodes, mollusques, escargots et limaces.

6. Procédé selon la revendication 5, dans lequel ledit invertébré est un moustique.

7. Procédé selon l'une quelconque des revendications 1 à 3, dans lequel ledit animal est un vertébré.

8. Procédé selon la revendication 7, dans lequel ledit vertébré est sélectionné parmi le groupe consistant aux rongeurs, lagomorphes, insectivores, taupes et musaraignes, chiroptères, carnivores, belettes, coyotes, ours, chiens et chats, artiodactyles, perissodactyles, primates, autres mammifères, reptiles, vertébrés marins comprenant les agnathies, chondrichtyens, requins, osteichthyens, oiseaux et pigeons.
